# EUROPEAN PATENT APPLICATION

(11) **EP 4 516 781 A1**
(43) Date of publication of application: **05.03.2025**
(21) Application number: 23795540.6
(22) Date of filing: 27.04.2023
(51) Int. Cl.: C07D 275/04, C07D 401/04, C07D 403/04, C07D 405/04, C07D 405/14, C07D 409/04, A61P 19/06, A61K 31/4155, A61K 31/416, A61K 31/4192, A61K 31/4439

(54) **COMPOUND FOR REDUCING URIC ACID**

(30) Priority: 27.04.2022 CN 202210456939
(71) Applicant: Atom Therapeutics Co., Ltd., Hangzhou, Zhejiang 310018 (CN)
(72) Inventor: SHI, Dongfang, Suzhou, Jiangsu 215123 (CN); FU, Zhangjin, Suzhou, Jiangsu 215123 (CN); YANG, Yan, Suzhou, Jiangsu 215123 (CN); LI, Haiming, Suzhou, Jiangsu 215123 (CN); YUAN, Chengyi, Suzhou, Jiangsu 215123 (CN)
(74) Representative: Cabinet Beau de Loménie
(86) International application number: PCT/CN2023/091140
(87) International publication number: WO 2023/208108

(57) **Abstract**

The invention discloses a class of compounds for reducing uric acid, which are compounds represented by formula (I) or their pharmaceutically acceptable salts. The compounds can significantly reduce the serum uric acid level in the rat model of hyperuricemia, and have potential application value in aspects such as anti-gout medicaments and anti-hyperuricemia medicaments.

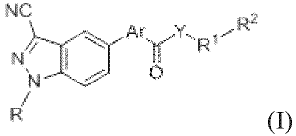

## Description

### TECHNICAL FIELD

The invention belongs to the technical field of medicine, and particularly relates to the compounds useful for reducing uric acid.

### BACKGROUND

Xanthine oxidase (XO) is an important target for drug therapy of hyperuricemia and gout. Hypoxanthine in human bodies is metabolized to xanthine, and xanthine is further metabolized to produce uric acid. In the forming process of uric acid, XO plays a decisive role, and thus when the activity of XO is inhibited, the conversion among hypoxanthine, xanthine and uric acid will be inhibited, thereby reducing the concentration of uric acid in human serum. Thus, inhibiting the activity of XO becomes the key to inhibiting uric acid production.

Generally, hyperuricemia is diagnosed when the serumuric acid level exceeds 420 µmol/L in men and exceeds 360 µmol/L in women. At present, hyperuricemia becomes the second major metabolic disease secondary to diabetes, seriously threatening human health. Hyperuricemia is not only an important biochemical basis for causing gout, but also is closely related to occurrence of hypertension, hyperlipidemia, atherosclerosis, obesity, and insulin resistance. According to data from the National Health and Nutrition Examination Survey, the prevalence of gout in the adult population in the United States was 3.9% (about 8.3 million people) between 2007 and 2008 (Zhu Y, Pandya BJ, Choi HK. Prevalence of Gout and Hyperuricemia in the US General Population: The National Health and Nutrition Examination Survey 2007-2008[J]. Arthritis Rheum, 2011, 63(10): 3136-3141); Meta analysis showed an overall prevalence of hyperuricemia in China of 13.3%, with gout being 1.1% (Liu R, Han C, Wu D, et al., Prevalence of hyperuricemia and gout in mainland China from 2000 to 2014: A systematic review and meta-analysis[J]. Biomed Research International, 2015: 1-12). Over the past few decades, the incidence of gout has also increased gradually due to the prevalence of combined diseases that can promote hyperuricemia, such as hypertension, obesity, metabolic syndrome, type 2 diabetes, and chronic kidney disease (Khanna D, Fitzgerald JD, Khanna PP, et al., American College of Rheumatology Guidelines for Management of Gout. Part 1: Systematic Nonpharmacologic and Pharmacologic Therapeutic Approaches to Hyperuricemia [J]. Arthritis Care & Research, 2012, 64(10): 1432-1446).

The increasing incidence of hyperuricemia and gout seriously endangers human health and there is need for early intervention and treatment. However, currently, there are very limited uric acid-lowering medicaments on the market, and they have significant toxic side effects and poor patient compliance. Currently, there are mainly three major classes of drugs for treating hyperuricemia and gout: urate anion transporter 1 (URAT1) inhibitors, xanthine oxidase inhibitors and urate oxidases.

The URAT1 inhibitors mainly act on the urate transporter of the renal proximal convoluted tubule, inhibiting the reabsorption of uric acid and increasing the its excretion, thereby reducing the concentration of uric acid in the body. Such drugs include Benzbromarone, Lesinurad and Probenecid. Benzbromarone is an effective uricosuric drug and has been marketed in many countries,buthas not been approved in the US. Benzbromarone was withdrawn from some countries in Europe in 2003 due to severe liver toxicity. Since there is an ethnic difference in liver-related adverse events related to Benzbromarone, Guideline for the diagnosis and management of hyperuricemia and gout in China (2019) recommend that Benzbromarone is a first-line uric acid-lowering drug. Lesinurad was marketed in the US in 2015, and its drug instruction contains a black box warning that it may cause acute renal failure and cardiovascular diseases (potentially fatal), and its efficacy is far inferior to that of Benzbromarone, and it must be used in combination with allopurinol. Probenecid is the first choice of uricosuric drugs in monotherapy uric acid-lowering treatment in the American guidelines. However, its application is limited due to the significant interactions with some commonly used drugs (such as non-steroidal anti-inflammatory drugs, beta-lactam drugs, and heparin).

Xanthine oxidase inhibitors include mainly allopurinol and febuxostat. Allopurinol has been widely used clinically since it was marketed by the FDA in 1966. Currently, the drug is still recommended as a first-line treatment for gout in most national gout guidelines. However, allopurinol has a poor therapeutic effect, only inhibiting reduced XO, and not affecting oxidized XO. A relevant research showed that even if allopurinol was used at the maximum dose, the rate of subjects reaching the treatment endpoint was less than 50% (Robert M, Douglas CA, Scott B. Less than half of patients treated with high-Dose allopurinol reach serum urea acid target[J]. ACR/ARHP Annual Meeting, 2017, Abstract Number: 1120). It also causes rash and other rare but deadly side reactions, including Stevens-Johnson syndrome and toxic epidermal necrolysis, and the mortality rate is about 10% to 30% (Bocquet H, Bagot M, Roujeau JC. Drug-induced pseudolymphoma and drug hypersensitivity syndrome (drug rash with eosinophilia and synergistic systems: DRESS [J]. Seminars in Cutaneous Medicine and Surgery, 1996, 15(4): 250-257). Allopurinol has been reported clinically to cause acute liver injury, and should be used with caution in the treatment of hyperuricemia patients with concurrent liver disease (Imai H, Kamei H, Onishi Y, et al., Successful living-donor liver transplantation for cholestatic liver failure induced by allopurinol: case report[J]. Transplantation Proceedings, 2015, 47(9): 2778-2781). Other side effects caused by allopurinol include gastric upset, nausea, abdominal pain, diarrhea, leukopenia and thrombocytopenia, headache, fever, loss of appetite, weight loss, pain in urination, hematuria, pruritus and lethargy.

Febuxostat is a non-purine XO inhibitor developed by Teijin in Japan, and can inhibit the reduced XO and oxidized XO, and the activity is obviously higher than that of allopurinol. Febuxostat was launched in Europe in 2008 and in the US in 2009. With the continuous expansion of the clinical application of Febuxostat, cardiovascular adverse reactions occurring when treating hyperuricemia are increasingly reported, and due to the cardiovascular toxicity (such as the risk of sudden death) thereof, the FDA required the addition of a black box risk warning to its drug instructions and adjusted the prescription information in 2019, changing it from a first-line drug to a second-line drug. In March 2018, the New England Journal of Medicine published a study of 6,190 gout patients, and the researchers found that after an average of 32 months of treatment, the overall risk of adverse cardiovascular events in the Febuxostat and allopurinol treatment groups was similar (HR 1.03, 95% CI, 0.87-1.23), but the whole cause mortality and cardiovascular death rate were higher for the Febuxostat group than for the allopurinol group. The patients in Febuxostat group had a 34% increase in cardiovascular mortality (HR 1.34, 95% CI, 1.03-1.73) and a 22% increase in all-cause mortality (HR 1.22, 95% CI, 1.01-1.47). Sudden cardiac death was the most common cause of cardiovascular death, with 83 cases (2.7%) in the Febuxostat group and 56 cases (1.8%) in the allopurinol group (William B, Kenneth G, Michael A, et al., Cardiovascular safety of Febuxostat or allopurinol in patients with gout [J]. The New England Journal of Medicine, 2018, 378: 1200-1210) . In addition, Febuxostat may also cause serious gastrointestinal tract toxic side effects, renal toxic side effects, liver dysfunction, etc.

Pegloticase is a mainly marketed urate oxidase drug at present, and is administrated by intravenous injection. The FDA has issued multiple black box warnings for this drug, with 20%-40% of patients experiencing severe immune allergic side effects. Its efficacy is average, with only 47% of patients reaching the treatment endpoint of below 0.36mmol/L.

In the past few decades, the development progress of therapeutic drugs for hyperuricemia and gout has been slow, but with the increasing incidence, the development of therapeutic drugs has attracted more and more attentions of researchers. The design of a new drug targeting xanthine oxidase has also received widespread attention, and various compounds have entered clinical trials, but they still face many problems such as significant toxic side effects, which require further research. Thus, highly efficient and low-toxicity XO inhibitors have great developmental potential and application value.

### SUMMARY

The purpose of the invention is to provide a compound having xanthine oxidase inhibitory activity on the basis of the prior art.

Another object of the invention is to provide use of said compound in the medical field.

The object of the invention can be achieved by the following measures: A compound as represented by general formula (I) or a pharmaceutically acceptable salt thereof, 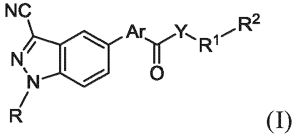 wherein,
R is C₁₋₆ alkyl, substituted C₁₋₆ alkyl, C₃₋₆ cycloalkyl, substituted C₃₋₆ cycloalkyl, C₃₋₆ heterocycloalkyl, or substituted C₃₋₆ heterocycloalkyl; wherein the substituent in each group involved in R is one or more selected from deuterium, cyano, nitro, halo, C₁₋₆ alkyl, C₁₋₆ alkoxy, C₃₋₆ cycloalkyl, or C₃₋₆ heterocycloalkyl;
Ar is a substituted or unsubstituted group of: 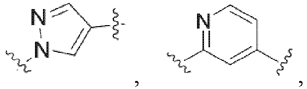 or 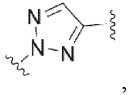 and the substituent in the Ar group is one or more selected from deuterium, hydroxyl, halo, C₁₋₄ alkyl, or C₁₋₄ alkoxy;
Y is O or NR³,
R¹ is a linking bond or substituted or unsubstituted C₁₋₆ alkylene or substituted or unsubstituted C₂₋₁₂ alkenylene, and the substituent in the R¹ group is one or more selected from deuterium, hydroxyl, amino, cyano, halo, C₁₋₄ alkyl, or C₁₋₄ alkoxy;
R² is one or more of hydrogen, nitrooxy, carboxyl, or the following substituted or unsubstituted groups: dioxol-2-one, C₄₋₁₂ fused heteroarylcyclyl, C₄₋₁₆ fused heteroarylcyclylpyrazolylcarbonyloxy, C₄₋₁₆ fused heteroarylcyclylpyridylcarbonyloxy, C₄₋₁₆ fused heteroarylcyclyltriazolylcarbonyloxy, C₂₋₆ ester, pyridyl, phenyl, C₁₋₆ alkoxy, C₂₋₂₀ alkenyl, C₂₋₂₀ alkynyl, C₂₋₈ alkylcarbonyloxy, or C₂₋₈ alkoxycarbonyloxy, and the substituent in the R² group is one or more selected from deuterium, hydroxyl, amino, cyano, halo, C₁₋₆ alkyl, halo C₁₋₆ alkyl, nitrooxy-substituted C₁₋₆ alkyl, or C₁₋₆ alkoxy; and
R³ is hydrogen or C₁₋₆ alkyl.

In a preferred embodiment, Ar is a substituted or unsubstituted group of: 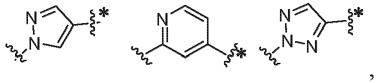 wherein "*" is an attachment site to C=O.

In a preferred embodiment, the compound of the invention is selected from compounds as represented by formula (II), (III) or (IV):

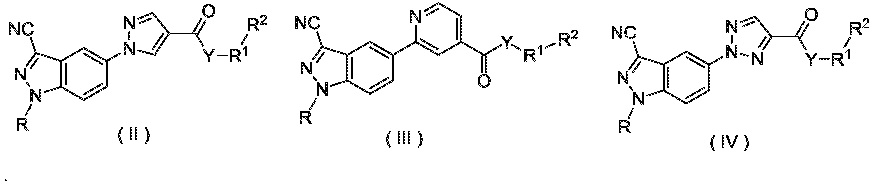

In a preferred embodiment, Y is O or NH.

In a preferred embodiment, R is C₃₋₆ alkyl, substituted C₁₋₆ alkyl, C₃₋₆ cycloalkyl, substituted C₃₋₆ cycloalkyl, C₃₋₆ heterocycloalkyl or substituted C₃₋₆ heterocycloalkyl; wherein the substituent in each group involved in R is one or more selected fromdeuterium, cyano, nitro, halo, C₁₋₅ alkyl, C₁₋₅ alkoxy, or C₃₋₆ cycloalkyl.

In a preferred embodiment, R is C₃₋₆ alkyl, substituted C₁₋₆ alkyl, C₃₋₆ cycloalkyl, substituted C₃₋₆ cycloalkyl, tetrahydrofuran, substituted tetrahydrofuran, tetrahydrothiophene, substituted tetrahydrothiophene, tetrahydropyrrole or substituted tetrahydropyrrole; wherein the substituent in each group involved in R is one or more selected fromdeuterium, cyano, nitro, halo, C₁₋₃ alkyl, C₁₋₅ alkoxy, or C₃₋₆ cycloalkyl.

In a preferred embodiment, R is C₃₋₆ alkyl, substituted C₁₋₃ alkyl, C₃₋₆ cycloalkyl or substituted C₃₋₆ cycloalkyl, wherein the substituent in the R group is selected from deuterium, halogen, or C₃₋₆ cycloalkyl.

In a preferred embodiment, R is C₃₋₆ alkyl or C₃₋₆ cycloalkyl.

In a preferred embodiment, R is n-propyl, isopropyl, n-butyl, isobutyl, cyclopropyl, cyclobutyl, or cyclopentyl.

In a preferred embodiment, R¹ is a linking bond or substituted or unsubstituted C₁₋₄ alkylene or substituted or unsubstituted C₄₋₁₂ alkenylene, and the substituent in the R¹ group is one or more selected from deuterium, amino, cyano, halogen, or C₁₋₄ alkoxy.

In a preferred embodiment, R² is one or more of hydrogen, nitrooxy, carboxyl, or the following substituted or unsubstituted groups: dioxol-2-one, indazolyl, quinolinyl, isoquinolinyl, indolyl, benzofuranyl, purinyl, indazolylpyrazolylcarbonyloxy, quinolinylpyrazolylcarbonyloxy, isoquinolinylpyrazolylcarbonyloxy, indolylpyrazolylcarbonyloxy, benzofuranylpyrazolylcarbonyloxy, purinylpyrazolylcarbonyloxy, indazolylpyridylcarbonyloxy, quinolinylpyridylcarbonyloxy, isoquinolinylpyridylcarbonyloxy, indolylpyridylcarbonyloxy, benzofuranylpyridylcarbonyloxy, purinylpyridylcarbonyloxy, indazolyltriazolylcarbonyloxy, quinolyltriazolylcarbonyloxy, isoquinolyltriazolylcarbonyloxy, indolyltriazolylcarbonyloxy, benzofuranyltriazolylcarbonyloxy, purinyltriazolylcarbonyloxy, C₂₋₆ ester, pyridyl, phenyl, C₁₋₆ alkoxy, C₆₋₂₀ alkenyl, C₆₋₂₀ alkynyl, C₂₋₈ alkylcarbonyloxy, or C₂₋₈ alkoxycarbonyloxy, and the substituent in the R² group is one or more selected from deuterium, hydroxyl, amino, cyano, halo, C₁₋₆ alkyl, halo C₁₋₆ alkyl, nitrooxy-substituted C₁₋₆ alkyl, or C₁₋₆ alkoxy

In a preferred embodiment,R² is one or more of hydrogen, nitrooxy, carboxyl, or the following substituted or unsubstituted groups: dioxol-2-one, indazolylpyrazolylcarbonyloxy, indazolylpyridylcarbonyloxy, indazolyltriazolylcarbonyloxy, indolylpyrazolylcarbonyloxy, indolylpyridylcarbonyloxy, indolyltriazolylcarbonyloxy, C₂₋₆ester, pyridyl, phenyl, C₁₋₆alkoxy, C₆₋₂₀alkenyl, C₂₋₈alkylcarbonyloxy, or C₂₋₈alkoxycarbonyloxy, and the substituent in the R² group is one or more selected from deuterium, hydroxyl, amino, cyano, halo, C₁₋₆ alkyl, nitrooxy-substituted C₁₋₆ alkyl, or C₁₋₆ alkoxy.

In a preferred embodiment, R³ is hydrogen, methyl, ethyl, n-propyl, isopropyl, or butyl.

In a preferred embodiment, the compound of the invention may be selected from:

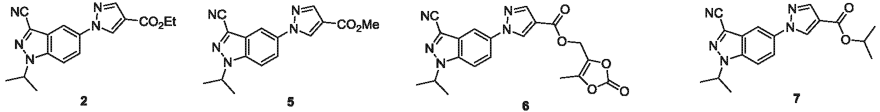

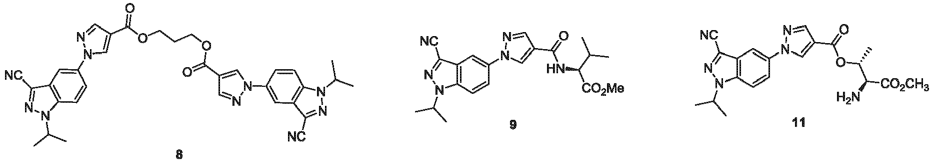

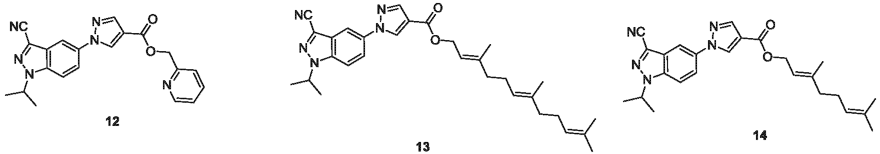

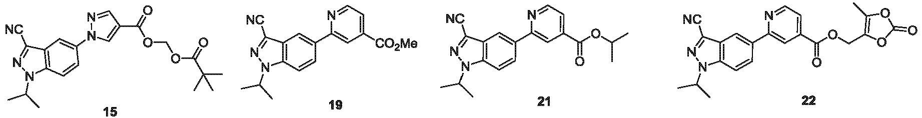

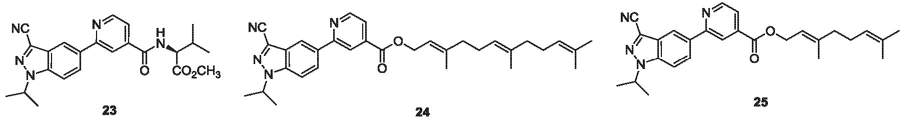

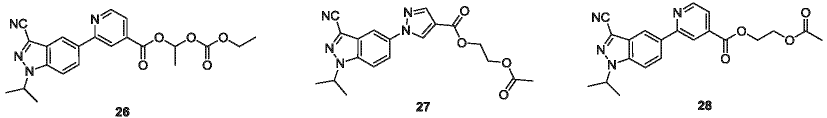

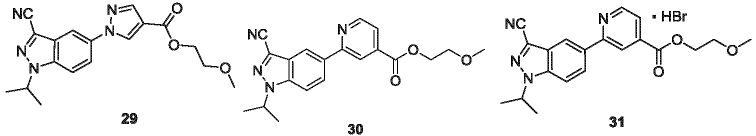

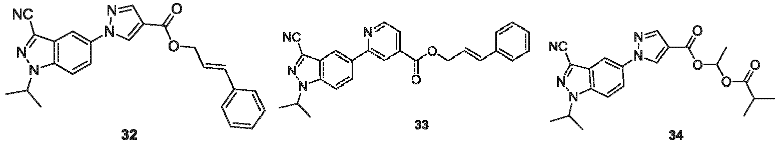

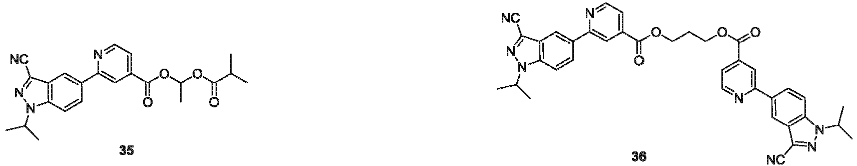

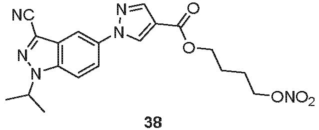

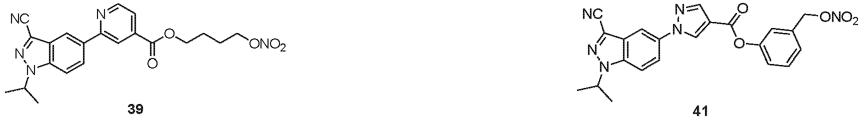

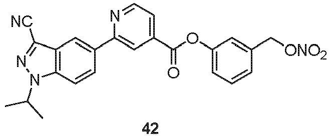

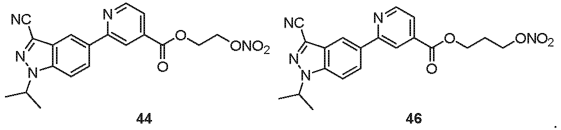

The invention also relates to a pharmaceutical composition, which uses the compound or the pharmaceutically acceptable salt thereof involved in the present application as an active substance, and uses a pharmaceutically acceptable excipient.

The compound or the pharmaceutically acceptable salt thereof of the invention can be used in the preparation of a xanthine oxidase inhibitor medicament, particularly in the preparation of an anti-gout medicament or an anti-hyperuricemia medicament.

The groups specified in the invention, if not explicitly defined otherwise, have the following meanings.

"H", i.e. hydrogen, refers to protium (1H), which is the main stable isotope of hydrogen element.

"D", or "deuterium", refers to a stable isotope of hydrogen, also known as heavy hydrogen, with the element symbol D.

"Halogen" refers to fluorine atom, chlorine atom, bromine atom, or iodine atom.

"Hydroxy" refers to -OH group.

"Amino" refers to -NH₂ group.

"Alkyl" refers to saturated aliphatic hydrocarbon groups containing 1 to 10 carbon atoms, including linear and branched chain groups (the numerical range mentioned in the present application, such as "1-10", means that the group, in this case alkyl, may contain 1 carbon atom, 2 carbon atoms, 3 carbon atoms, and so on, up to10 carbon atoms). Alkyl containing 1-4 carbon atoms is referred to as lower alkyl. When the lower alkyl has no substituent, it is called unsubstituted lower alkyl. The alkyl may be selected from C₁₋₆ alkyl, C₁₋₅ alkyl, C₁₋₄ alkyl, C₁₋₃ alkyl, C₁₋₂ alkyl, C₂₋₃ alkyl, C₂₋₄ alkyl, etc. Specific alkyl includes, but is not limited to, methyl, ethyl, propyl, 2-propyl, n-butyl, isobutyl, t-butyl, etc. The alkyl may be substituted or unsubstituted.

"Alkenyl" refers to hydrocarbon groups having one or more "C=C" and containing 2-30 carbon atoms, including both linear and branched chain groups (the numerical range mentioned in the present application, such as "2-10", means that the group, in this case alkenyl, may contain 2 carbon atoms, 3 carbon atoms, and so on, up to10 carbon atoms). The alkenyl may be selected from C₂₋₂₀ alkenyl, C₂₋₁₈ alkenyl, C₂₋₁₆ alkenyl, C₂₋₁₄ alkenyl, C₂₋₁₂ alkenyl, C₄₋₁₄ alkenyl, C₄₋₁₂ alkenyl, etc. Specific alkenyl includes, but is not limited to, ethenyl, propenyl, allyl, butenyl, isobutenyl, t-butenyl, 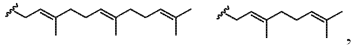 etc.

"Alkoxy" represents an-O-(unsubstituted alkyl) and-O-(unsubstituted cycloalkyl) group, and further represents-O-(unsubstituted alkyl). The alkyl therein may be selected from C₁₋₆ alkyl, C₁₋₅ alkyl, C₁₋₄ alkyl, C₁₋₃ alkyl, C₁₋₂ alkyl, C₂₋₃ alkyl, C₂₋₄ alkyl, etc. Representative examples include, but are not limited to, methoxy, ethoxy, propoxy, cyclopropoxy, etc.

"Dioxol-2-one" is a 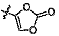 group.

"Pyrazolyl" refers to any one of

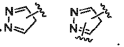

"Triazolyl" includes 1,2,3-triazolyl, wherein "1,2,3-triazolyl" is

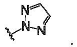

"Pyridyl" refers to any one of

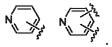

"Fused heteroarylcyclyl" refers to an aromatic group containing two or more fused rings and heteroatoms, including but not limited to indazolyl, quinolinyl, isoquinolinyl, indolyl, benzofuranyl, purinyl, acridinyl, etc.

"Carboxy" refers to -COOH group.

"Ester group" refers to a "-C(=O)-O-alkyl" group, wherein the alkyl may be selected from C₁₋₆ alkyl, C₁₋₅ alkyl, C₁₋₄ alkyl, C₁₋₃ alkyl, C₁₋₂ alkyl, C₂₋₃ alkyl, C₂₋₄ alkyl, etc. Representative examples include, but are not limited to, methyl formate group, ethyl formate group, n-propyl formate group, isopropyl formate group,etc. The substituted ester group means that hydrogen in the ester group is substituted with one substituent, or a plurality of hydrogen in the ester group are respectively substituted with same or different substituents.

"Heterocycloalkyl" refers to a saturated cyclic group containing 3-10 ring atoms, with one or more are heteroatoms selected from N, O, and S. The numerical range mentioned in the present application, such as "3-6", means that the group, in this case heterocycloalkyl, may contain 3 carbon atoms, 4 carbon atoms, 5 carbon atoms, and so on, up to6 carbon atoms as ring atoms. The heterocycloalkyl can be selected from C₃₋₈ heterocycloalkyl, C₃₋₆ heterocycloalkyl, C₃₋₅ heterocycloalkyl, C₃₋₄ heterocycloalkyl, C₃₋₉ heterocycloalkyl, C₄₋₆ heterocycloalkyl, etc. Specific alkyl includes, but is not limited to, tetrahydrofuran, tetrahydropyrrole, tetrahydrothiophene, 1,4-dioxane, oxospiro[3,3]heptanyl, oxospiro[4,4]nonanyl, oxospiro[5,5]undecanyl, oxospiro[6,6]tridecyl, oxobicyclo[1,1,1]pentanyl, oxobicyclo[2,2,2]octanyl, oxobicyclo[3,2,1]octanyl, azaspiro[3,3]heptanyl, azaspiro[4,4]nonanyl, azaspiro[5,5]undecanyl, azaspiro[6,6]tridecyl, azabicyclo[1,1,1]pentanyl, azabicyclo[2,2,2]octanyl, azabicyclo[3,2,1]octanyl, etc. The heterocycloalkylmay be substituted or unsubstituted.

"C₄₋₁₆ fused heteroarylpyrazolylcarbonyloxy" refers to an -O-C(=O)-pyrazolyl-fused heteroarylcyclyl group containing 4-16 carbon atoms. Specific examples include, but are not limited to, indazolylpyrazolylcarbonyloxy 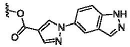 and indolylpyrazolylcarbonyloxy

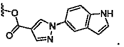

"C₂₋₈ alkoxycarbonyloxy" refers to an -O-C(=O)-O-alkyl group containing 2-8 carbon atoms.

"C₂₋₈ alkylcarbonyloxy" refers to an -O-C(=O)-alkyl group containing 2-8 carbon atoms.

"C₄₋₁₆ fused heteroarylcyclylpyridylcarbonyloxy" refers to an -O-C(=O)-pyridyl-fused heteroarylcyclyl group containing 4-16 carbon atoms. Specific examples include, but are not limited to, indazolylpyridylcarbonyloxy 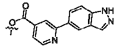 and indolylpyridylcarbonyloxy

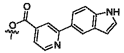

"C₄₋₁₆fused heteroaryltriazolylcarbonyloxy" refers to an -O-C(=O)-triazolyl-fused heteroarylcyclyl group containing 4-16 carbon atoms. Specific examples include, but are not limited to, indazolyltriazolylcarbonyloxy 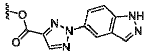 and indolyltriazolylcarbonyloxy

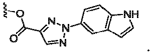

"Linking bond" means that groups on two ends are directly linked by a covalent bond. Taking the group fragment Y-R¹-R² as an example, when R¹ is a linking bond, the group fragment is Y-R².

"Nitrooxy" refers to-ONO₂ group.

"Pharmaceutically acceptable salt" is a salt formed by the compound of general formula (I)with an organic or inorganic acid, and represents those salts that retain the biological effectiveness and properties of the parent compound. Such salts include, but are not limited to:
(1) salts formed with an acid, obtained by reaction of a free base of the parent compound with an inorganic acid or an organic acid, wherein the inorganic acid is, for example, but not limited to, hydrochloric acid, hydrobromic acid, nitric acid, phosphoric acid, metaphosphoric acid, sulfuric acid, sulfurous acid, perchloric acid, or the like; and the organic acid is, for example, but not limited to, acetic acid, propionic acid, acrylic acid, oxalic acid, ("D") or ("L") malic acid, fumaric acid, maleic acid, hydroxybenzoic acid, γ-hydroxybutyric acid, methoxybenzoic acid, phthalic acid, methanesulfonic acid, ethanesulfonic acid, naphthalene-1-sulfonic acid, naphthalene-2-sulfonic acid, p-toluenesulfonic acid, salicylic acid, tartaric acid, citric acid, lactic acid, mandelic acid, succinic acid, or malonic acid, or the like; and
(2) salts formed when an acidic proton in the parent compound is replaced by a metal ion or the parent compound is coordinated with an organic base, wherein the metal ion, is, for example, an alkali metal ion, an alkaline earth metal ion, or an aluminum ion, and the organic base is, for example, ethanolamine, diethanolamine, triethanolamine, tromethamine, N-methylglucamine, or the like.

"Pharmaceutical composition" refers to a mixture of one or more compounds described herein, or a pharmaceutically acceptable salt and a prodrug thereof, with other chemical ingredients, such as a pharmaceutically acceptable carrier and an excipient. The purpose of the pharmaceutical composition is to facilitate administration of the compound to an organism.

The invention further sets forth a pharmaceutical composition comprising any one of the compounds described above, a pharmaceutically acceptable salt thereof or a hydrolyzable prodrug thereof, together with other pharmaceutically active ingredients.

In the invention, any of the described compounds and pharmaceutically acceptable salts thereof can be formulated into any dosage form which is clinically or pharmaceutically acceptable in a manner known in the art. For oral administration, conventional solid formulations such as tablets, capsules, pills, granules, etc. can be prepared; oral liquid formulations such as oral solutions, oral suspensions, syrups, etc. can also be prepared. When preparing oral formulations, suitable fillers, binders, disintegrating agents, lubricants, etc. can be added. For parenteral administration, injections can be prepared, including injections, sterile powders for injection and concentrated solutions for injection. When preparing injections, conventional methods in the existing pharmaceutical field can be used, and when preparing the injections, additives may not be added, or appropriate additives may be added according to the properties of the drug.

The compounds provided in the invention can significantly reduce the serum uric acid level in a rat model of hyperuricemia, and has potential application value in aspects such as anti-gout medicaments and anti-hyperuricemia medicaments. Because Febuxostat can cause severe sudden cardiac death, severe renal toxicity and liver toxicity, the compounds provided by the present invention may have certain advantages in reducing drug toxicity and have good prospects for drug development.

### DETAILED DESCRIPTION

The invention is further described below with reference to embodiments, however, the scope of protection of the invention is not limited to the following embodiments.

### Example 1: Synthesis of ethyl

### 1-(3-cyano-1-isopropyl-1H-indazol-5-yl)-1H-pyrazole-4-carboxylate (2)

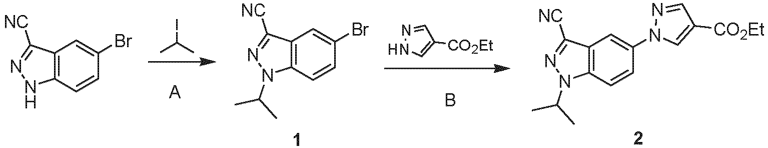

Step A: A mixture comprising 5-bromo-1H-indazole-3-carbonitrile (3.0 g, 13.5 mmol), iodoisopropane (9.19 g, 54.1 mmol), cesium carbonate (8.80 g, 27.0 mmol) and DMF (50 mL) was stirred at 80°C for 1.5 hour, cooled to room temperature, and filtered to remove insolubles. Water (200 mL) was added, and ethyl acetate (80 mL x 3) was added for extraction. The combined organic phase was washed successively with water (50 mL x 2) and saturated brine (50 mL), and dried over anhydrous sodium sulfate. The solvent was evaporated under reduced pressure. The product was purified by column chromatography (200-300 mesh silica gel, ethyl acetate: petroleum ether = 1:50-1:30 elution) to give 5-bromo-1-isopropyl-1H-indazole-3-carbonitrile (**1**) (2.10 g). The yield was 58.9%. ¹H NMR (CDCl₃,400 MHz): δ 7.95 (d, J = 1.2 Hz,1H),7.55 (dd, J = 1.2,8.8 Hz,1H),7.45 (d, J = 8.8 Hz,1H),4.93-4.87 (m,1H),1.61 (d, J = 6.4 Hz,6H).

Step B: A mixture comprising ethyl 1H-pyrazole-4-carboxylate (1.06 g, 7.56 mmol), compound 1 (1.0 g, 3.79 mmol), potassium carbonate (833 mg, 6.04 mmol), copper iodide (1.05 g, 5.51 mmol), (1S,2S)-1,2-diaminocyclohexane (432 mg, 3.78 mmol), and DMF (20 mL) was stirred under nitrogen at 110°C overnight,and cooled to room temperature. Water (80 mL) was added. Ethyl acetate (40 mL x 3) was used for extraction. The combined organic phase was washed with saturated brine (30 mL x 3), and dried over anhydrous sodium sulfate. The solvent was evaporated under reduced pressure. The product was purified by column chromatography (200-300 mesh silica gel, ethyl acetate: petroleum ether = 1: 15-1:4 elution) to give ethyl 1-(3-cyano-1-isopropyl-1H-indazol-5-yl)-1H-pyrazole-4-carboxylate (**2**) (500 mg). The yield was 40.8%. ¹H NMR (DMSO-d₆,400 MHz): δ 9.31 (s,1H),8.47 (d, J = 1.2 Hz,1H),8.23-8.16 (m,3H),5.29-5.22 (m,1H),4.29 (q, J = 7.2 Hz,2H),1.54 (d, J = 7.2 Hz,6H),1.33 (t, J = 7.2 Hz,3H). MS (ESI, m/z):324.1 [M+H]⁺.

### Example 2: Synthesis of methyl

### 1-(3-cyano-1-isopropyl-1H-indazol-5-yl)-1H-pyrazole-4-carboxylate (5)

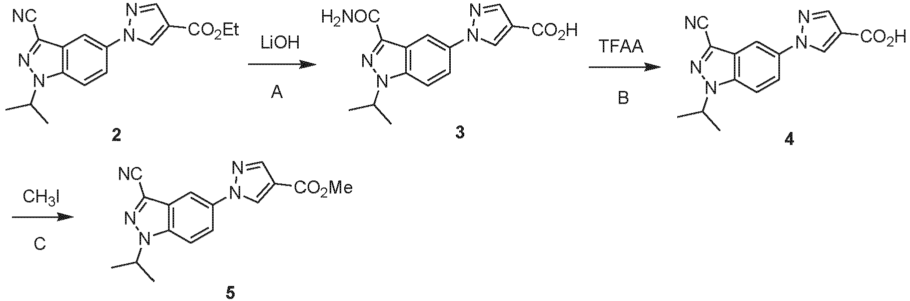

Step A: A mixture comprising compound **2** (500 mg, 1.55 mmol), lithium hydroxide hydrate (623 mg, 14.8 mmol), water (1.5 mL), methanol (1.5 mL), and THF (1.5 mL) was stirred at 20°C for 2 hours. A portion of the solvent was evaporated under reduced pressure. Water (8 mL) was added. The pH was adjusted to 1-2 by 6 M hydrochloric acid. The filter cake was recrystallized in acetonitrile to give 1-(3-carbamoyl-1-isopropyl-1H-indazol-5-yl)-1H-pyrazole-4-carboxylic acid (3)(300 mg). The yield was 61.8%. MS (ESI, m/z):313.9 [M+H]⁺.

Step B: To a solution of compound **3** (300 mg, 0.958 mmol) in dichloromethane (5 mL), trifluoroacetic anhydride (906 mg, 4.31 mmol) and triethylamine (873 mg, 8.63 mmol) were added in an ice-water bath. After addition, the resulting mixture was stirred overnight at room temperature. Saturated brine (20 mL) was added. Dichloromethane (20 mL x 2) was added for extraction. The combined organic phase was washed with saturated brine (20 mL x 2), and dried over anhydrous sodium sulfate. The solvent was evaporated under reduced pressure. Then, purification was performed by preparative HPLC to give 1-(3-cyano-1-isopropyl-1H-indazol-5-yl)-1H-pyrazole-4-carboxylic acid (**4**). ¹H NMR (DMSO-d₆,400 MHz): δ 9.10 (s,1H),8.39 (d, J = 1.6 Hz,1H),8.21-8.12 (m,2H),8.05 (s,1H),5.22 (q, J = 6.4 Hz,1H),1.53 (d, J = 6.4 Hz,6H). MS (ESI, m/z):296.2 [M+H]⁺.

Step C: A mixture comprising compound **4** (250 mg, 0.847 mmol), iodomethane (192 mg, 1.35 mmol), potassium carbonate (235 mg, 1.70 mmol), and DMF (5 mL) was stirred overnight at room temperature. Water (20 mL) was added for filtration. The filter cake was purified by column chromatography (200-300 mesh silica gel, dichloromethane: petroleum ether = 1:1elution) to give methyl 1-(3-cyano-1-isopropyl-1H-indazol-5-yl)-1H-pyrazole-4-carboxylate (**5**). ¹H NMR (DMSO-d₆,400 MHz): δ 9.34 (s,1H),8.46 (d, J = 1.6 Hz,1H),8.23-8.16 (m,3H),5.28-5.22 (m,1H),3.83 (s,3H),1.54 (d, J = 7.2 Hz,6H). MS (ESI, m/z):310.1 [M+H]⁺.

### Example 3: Synthesis of (5-methyl-2-oxo-1,3-dioxol-4-yl) methyl

### 1-(3-cyano-1-isopropyl-1H-indazol-5-yl)-1H-pyrazole-4-carboxylate (6)

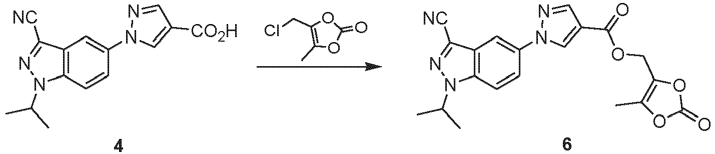

A mixture comprising compound **4** (150 mg, 0.508 mmol), 4-chloromethyl-5-methyl-1,3-dioxol-2-one (91 mg, 0.613 mmol), potassium carbonate (140 mg, 1.01 mmol), potassium iodide (110 mg, 0.663 mmol), and DMF (5 mL) was stirred at room temperature for 3 hours. Water (20 mL) was added for filtration. The filter cake was purified by column chromatography (200-300 mesh silica gel, dichloromethane elution) to give (5-methyl-2-oxo-1,3-dioxol-4-yl)methyl 1-(3-cyano-1-isopropyl-1H-indazol-5-yl)-1H-pyrazole-4-carboxylate (**6**). ¹H NMR (DMSO-d₆,400 MHz): δ 9.47 (s,1H),8.58 (d, J = 1.2 Hz,1H),8.34-8.25 (m,3H),5.38-5.31 (m,1H),5.29 (s,2H),2.33 (s,3H),1.63 (d, J = 6.8 Hz,6H). MS (ESI, m/z):408.1 [M+H]⁺.

### Example 4: Synthesis of isopropyl

### 1-(3-cyano-1-isopropyl-1H-indazol-5-yl)-1H-pyrazole-4-carboxylate (7)

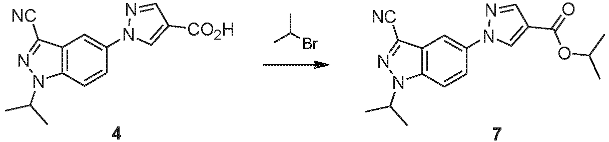

A mixture comprising compound **4** (250 mg, 0.847 mmol), bromoisopropane (325 mg, 2.64 mmol), potassium carbonate (235 mg, 1.70 mmol), potassium iodide (190 mg, 1.14 mmol), and DMF (5 mL) was stirred at 30°C for 48 hours. Water (20 mL) was added for filtration. The filter cake was purified by column chromatography (200-300 mesh silica gel, dichloromethane: petroleum ether = 1:1elution) to give isopropyl 1-(3-cyano-1-isopropyl-1H-indazol-5-yl)-1H-pyrazole-4-carboxylate (7) (190 mg). The yield was 66.5%. ¹H NMR (DMSO-d₆,400 MHz): δ 9.28 (s,1H),8.47 (d, J = 1.2 Hz,1H),8.24-8.16 (m,3H),5.29-5.22 (m,1H),5.16-5.10 (m,1H),1.54 (d, J = 6.4 Hz,6H),1.32 (d, J = 6.4 Hz,6H). MS (ESI, m/z):338.1 [M+H]⁺.

### Example 5: Synthesis of

### bis[1-(3-cyano-1-isopropyl-1H-indazol-5-yl)-1H-pyrazole-4-carboxylic acid]propane-1,3-diester (8)

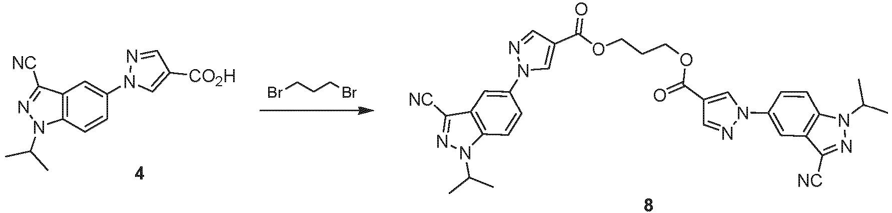

A mixture comprising compound **4** (200 mg, 0.677 mmol), 1,3-dibromopropane (68 mg, 0.337 mmol), potassium carbonate (187 mg, 1.35 mmol), potassium iodide (146 mg, 0.880 mmol), and DMF (5 mL) was stirred at 30°C for 48 hours. Water (20 mL) was added for filtration. The filter cake was purified by column chromatography (200-300 mesh silica gel, dichloromethane: petroleum ether = 1:1elution) to give bis[1-(3-cyano-1-isopropyl-1H-indazol-5-yl)-1H-pyrazole-4-carboxylic acid]propane-1,3-diester (**8**) (146 mg). The yield was 68.4%. ¹H NMR (DMSO-d₆,400 MHz): δ 9.20 (s,2H),8.24 (s,2H),8.12-8.05 (m,6H),5.23-5.16 (m,2H),4.45 (t, J = 6.0 Hz,4H),2.18 (t, J = 6.0 Hz,2H),1.53 (d, J = 6.4 Hz,12H). MS (ESI, m/z):631.1 [M+H]⁺.

### Example 6: Synthesis of

### [1-(3-cyano-1-isopropyl-1H-indazol-5-yl)-1H-pyrazole-4-carbonyl]-L-valine methyl ester (9)

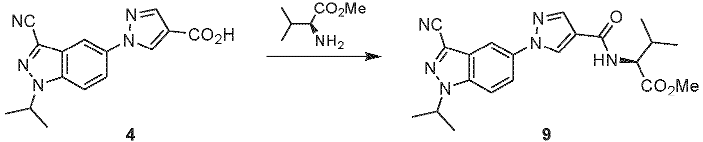

A mixture comprising compound **4** (200 mg, 0.677 mmol), L-valine methyl ester hydrochloride (136 mg, 0.811 mmol), diisopropylethylamine (219 mg, 1.69 mmol), HBTU (385 mg, 1.02 mmol), and DMF (5 mL) was stirred overnight at room temperature. Water (20 mL) was added. Ethyl acetate (30 mL x 2) was added for extraction. The combined organic phase was washed with saturated brine (15 mL x 3) and dried over anhydrous sodium sulfate. The solvent was evaporated under reduced pressure. The product was purified by column chromatography (200-300 mesh silica gel, petroleum ether: ethyl acetate: triethylamine = 100:10:1) to give [1-(3-cyano-1-isopropyl-1H-indazol-5-yl)-1H-pyrazole-4-carbonyl]-L-valine methyl ester (9) (268 mg). The yield was 96.9%. ¹H NMR (DMSO-d₆,400 MHz): δ 9.23 (s,1H),8.34-8.29 (m,3H),8.20-8.14 (m,2H),5.27-5.24 (m,1H),4.38-4.34 (m,1H),3.67 (s,3H),2.18-2.13 (m,1H),1.55 (d, J = 6.8 Hz,6H),0.99 (d, J = 6.8 Hz,3H),0.94 (d, J = 6.4 Hz,3H). MS (ESI, m/z):409.2 [M+H]⁺.

### Example 7: Synthesis of [(2R,3S)-3-amino-4-methoxy-4-oxobutan-2-yl]

### 1-(3-cyano-1-isopropyl-1H-indazol-5-yl)-1H-pyrazole-4-carboxylate (11)

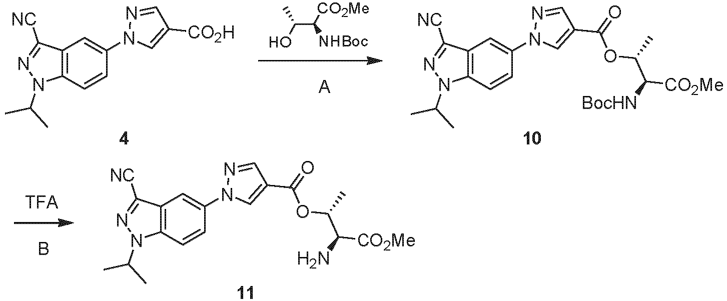

Step A: A mixture comprising compound **4** (200 mg, 0.677 mmol), Boc-L-threonine methyl ester (189 mg, 0.810 mmol), DCC (210 mg, 1.02 mmol), and dichloromethane (5 mL) was stirred overnight at room temperature. Insolubles were removed by filtration, and the filter cake was rinsed with dichloromethane (5 mL). The solvent was evaporated under reduced pressure. The product was purified by column chromatography (200-300 mesh silica gel, petroleum ether: ethyl acetate: triethylamine = 100:4:1) to give [(2R,3S)-3-(Boc-amino)-4-methoxy-4-oxobutan-2-yl]1-(3-cyano-1-isopropyl-1H-ind azol-5-yl)-1H-pyrazole-4-carboxylate (**10**)(340 mg). The yield was 99.8%.

Step B: A solution of compound **10** (340 mg, 0.666 mmol) and trifluoroacetic acid (0.3 mL) in dichloromethane (5 mL) was stirred at room temperature overnight. Water (20 mL) was added, and the pH was adjusted to 7-8 bya saturated sodium bicarbonate solution. Dichloromethane (20 mL x 2) was used for extraction. The combined organic phase was washed with saturated brine (10 mL x 2), and dried over anhydrous sodium sulfate. The solvent was evaporated under reduced pressure. The resulting product was recrystallized in ethyl acetate/petroleum ether to give [(2R,3S)-3-amino-4-methoxy-4-oxobutan-2-yl] 1-(3-cyano-1-isopropyl-1H-indazol-5-yl)-1H-pyrazole-4-carboxylate (11). ¹H NMR (DMSO-d₆,400 MHz): δ 9.32 (s,1H),8.44 (s,1H),8.23-8.18 (m,3H),5.32-5.23 (m,2H),3.61 (s,3H),3.55 (s,1H),2.06 (s,2H),1.54 (d, J = 6.4 Hz,6H),1.35 (d, J = 6.4 Hz,3H). MS (ESI, m/z):411.1 [M+H]⁺.

### Example 8: Synthesis of (pyridin-2-yl) methyl

### 1-(3-cyano-1-isopropyl-1H-indazol-5-yl)-1H-pyrazole-4-carboxylate (12)

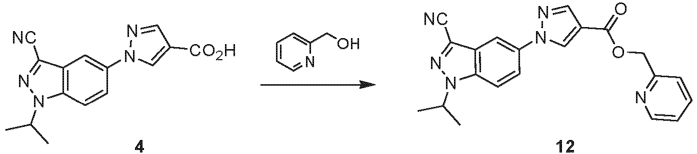

A mixture comprising compound **4** (190 mg, 0.643 mmol), pyridine-2-methanol (84 mg, 0.770 mmol), DCC (210 mg, 0.969 mmol), DMAP (2 mg, 0.0163 mmol), and dichloromethane (5 mL) was stirred overnight at room temperature. Insolubles were removed by filtration, and the filter cake was rinsed with dichloromethane (5 mL). The solvent was evaporated under reduced pressure. The product was purified by column chromatography (200-300 mesh silica gel, dichloromethane: triethylamine = 100:1) to give (pyridin-2-yl)methyl1-(3-cyano-1-isopropyl-1H-indazol-5-yl)-1H-pyrazole-4-carboxy late (12)(75 mg). The yield was 30.2%. ¹H NMR (DMSO-d₆,400 MHz): δ 9.42 (s,1H),8.58 (d, J = 4.4 Hz,1H),8.50 (d, J = 1.6 Hz,1H),8.30 (s,1H),8.23 (d, J = 1.6 Hz,1H),8.20 (s,1H),7.88-7.85 (m,1H),7.56 (d, J = 8.0 Hz,1H),7.39-7.36 (m,1H),5.40 (s,2H),5.29-5.23 (m,1H),1.54 (d, J = 6.4 Hz,6H). MS (ESI, m/z):387.1 [M+H]⁺.

### Example 9: (3,7,11-trimethyldodecyl-2,6,10-trien-1-yl)

### 1-(3-cyano-1-isopropyl-1H-indazol-5-yl)-1H-pyrazole-4-carboxylate (13)

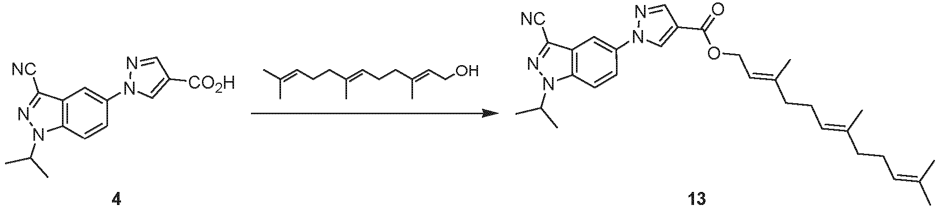

Compound **4** and 3,7,11-trimethyldodecyl-2,6,10-trien-1-ol were used as raw materials, and reference can be made to Example 8 for the experimental procedure for synthesizing compound **13.** ¹H NMR (DMSO-d₆,400 MHz): δ 9.29 (s,1H),8.47 (s,1H),8.23-8.15 (m,3H),5.44-5.40 (m,1H),5.29-5.22 (m,1H),5.10-5.00 (m,2H),4.79-4.74 (m,2H),2.11-1.89 (m,8H),1.83-1.74 (m,6H),1.58-1.52 (m,12H). MS (ESI, m/z):500.3 [M+H]⁺.

### Example 10: Synthesis of (3,7-dimethyloctane-2,6-dien-1-yl)

### 1-(3-cyano-1-isopropyl-1H-indazol-5-yl)-1H-pyrazole-4-carboxylate (14)

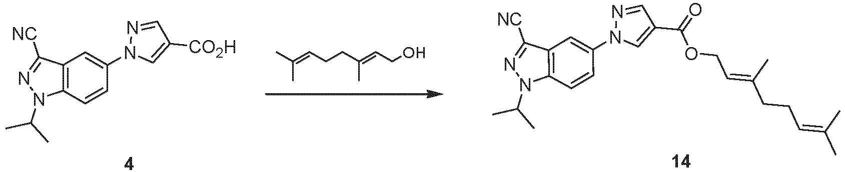

Compound **4** and 3,7-dimethyloctan-2,6-dien-1-ol were used as raw materials, and reference can be made to Example 8 for the experimental procedure for synthesizing compound **14.** ¹H NMR (DMSO-d₆,400 MHz): δ 9.31 (s,1H),8.47 (d, J = 1.6 Hz,1H),8.23-8.16 (m,3H),5.44-5.41 (m,1H),5.28-5.23 (m,1H),5.22-5.07 (m,1H),4.78 (d, J = 6.8 Hz,2H),2.10-2.03 (m,4H),1.74 (s,3H),1.63 (s,3H),1.57 (s,3H),1.55 (s,3H),1.53 (s,3H). MS (ESI, m/z):432.2 [M+H]⁺.

### Example 11: Synthesis of (pivaloyloxy)methyl

### 1-(3-cyano-1-isopropyl-1H-indazol-5-yl)-1H-pyrazole-4-carboxylate (15)

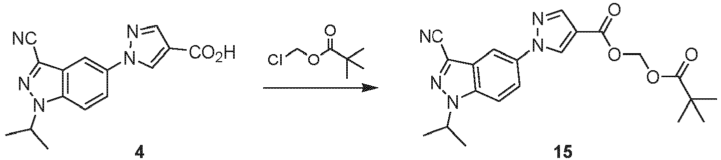

A mixture comprising compound **4** (120 mg, 0.406 mmol), chloromethyl pivalate (74 mg, 0.491 mmol), potassium carbonate (113 mg, 0.818 mmol), potassium iodide (88 mg, 0.530 mmol), and DMF (5 mL) was stirred at room temperature for 48 hours. Water (20 mL) was added. Ethyl acetate (20 mL x 2) was used for extraction. The combined organic phase was washed with saturated brine (20 mL x 2) and dried over anhydrous sodium sulfate. The solvent was evaporated under reduced pressure. The product was purified by column chromatography (200-300 mesh silica gel, dichloromethane: petroleum ether=1:1) to give (pivaloyloxy)methyl1-(3-cyano-1-isopropyl-1H-indazol-5-yl)-1H-pyrazole-4-carboxy late (15). ¹H NMR (DMSO-d₆,400 MHz): δ 9.41 (s,1H),8.50 (d, J = 1.2 Hz,1H),8.26-8.17 (m,3H),5.94 (s,2H),5.29-5.22 (m,1H),1.54 (d, J = 6.4 Hz,6H),1.17 (s,9H). MS (ESI, m/z):410.1 [M+H]⁺.

### Example 12: Synthesis of methyl

### 2-(3-cyano-1-isopropyl-1H-indazol-5-yl)isonicotinate (19)

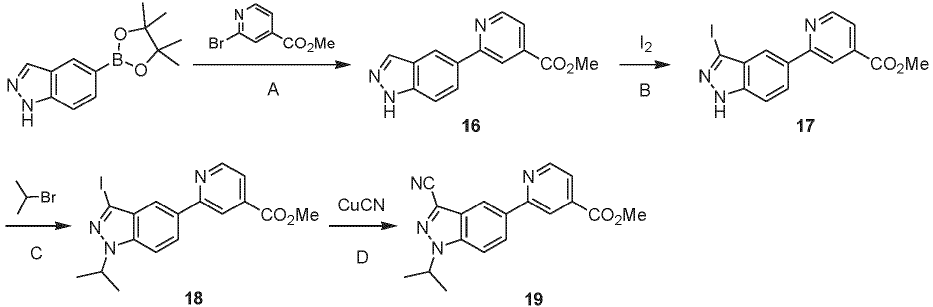

Step A: To a mixture comprising 5-(4,4,5,5-tetramethyl-1,3,2-dioxaboran-2-yl)-1H-indazole (8.40 g, 34.4 mmol), methyl 2-bromopyridine-4-carboxylate (9.0 g, 41.7 mmol), potassium carbonate (12.0 g, 87.0 mmol), dioxane (100 mL), and water (20 mL), [1,1'-bis(diphenylphosphino)ferrocene]palladium dichloride (1.20 g, 1.47 mmol) was added. After the addition was complete, the resulting mixture was stirred at 80°C for 3 hours under nitrogen, cooled to room temperature, and filtered. The filter cake was rinsed with a small amount of ethyl acetate. Most of the solvent was evaporated under reduced pressure. Ethyl acetate (500 mL) was added. The product was washed with saturated brine (100 mL), and dried over anhydrous sodium sulfate. The solvent was evaporated under reduced pressure, and the product was purified by column chromatography (200-300 mesh silica gel, ethyl acetate: dichloromethane = 1:15) to give methyl 2-(1H-indazol-5-yl)isonicotinate (**16**). ¹H NMR (DMSO-d₆,400 MHz): δ 13.25 (s,1H),8.90 (d, J = 4.8 Hz,1H),8.61 (s,1H),8.40 (s,1H),8.23-8.19 (m,2H),7.79 (dd, J = 1.2,4.8 Hz,1H),7.69 (d, J = 8.8 Hz,1H),3.99 (s,3H). MS (ESI, m/z):254.1 [M+H]⁺.

Step B: To a solution of compound **16** (2.75 g, 10.9 mmol) in DMF (30 mL), cesium carbonate (7.08 g, 21.7 mmol) and iodine (5.50 g, 21.7 mmol) were added. After the addition was complete, the resulting mixture was stirred at room temperature for 2 hours. Water (120 mL) and a 2M sodium thiosulfate solution (20 mL) were added. The filter cake was dissolved with ethyl acetate (300 mL). Insolubles were filtered. Then, the resultant was dried over anhydrous sodium sulfate. The solvent was evaporated under reduced pressure to give methyl 2-(3-iodo-1H-indazol-5-yl)isonicotinate (**17**) (3.90 g). The yield was 94.5%.

Step C: A mixture comprising compound **17** (3.90 g, 10.3 mmol), potassium carbonate (1.70 g, 12.3 mmol), bromoisopropane (1.90 g, 15.4 mmol), potassium iodide (340 mg, 2.05 mmol), and DMF (40 mL) was stirred overnight at 60°C, and cooled to room temperature. Water (160 mL) was added. Ethyl acetate (100 mL x 2) was used for extraction. The combined organic phase was washed successively with water (40 mL x 2) and saturated brine (40 mL), and dried over anhydrous sodium sulfate. The solvent was evaporated under reduced pressure. The product was purified by column chromatography (200-300 mesh silica gel, ethyl acetate: petroleum ether = 1:10) to give methyl 2-(3-iodo-1-isopropyl-1H-indazol-5-yl)isonicotinate (**18**) (3.81 g). The yield was 87.8%.

Step D: A mixture comprising compound **18** (3.81 g, 9.04 mmol), cuprous cyanide (1.14 g, 12.7 mmol), and DMF (30 mL) was stirred at 120°C overnight, and cooled to room temperature. Ethyl acetate (100 mL) and water (100 mL) were added. Insolubles were removed by filtration. The mixture was layered. The aqueous layer was extracted with ethyl acetate (100 mL x 2). The combined organic phase was washed with water (40 mL x 2) and saturated brine (40 mL), and dried over anhydrous sodium sulfate. The solvent was evaporated under reduced pressure. The product was purified by column chromatography (200-300 mesh silica gel, ethyl acetate: petroleum ether=1:12-1:2 elution) to give methyl 2-(3-cyano-1-isopropyl-1H-indazol-5-yl)isonicotinate (**19**) (1.0 g). The yield was 34.5%. ¹H NMR (DMSO-d₆,400 MHz): δ 8.92 (d, J = 4.8 Hz,1H),8.67 (s,1H),8.53 (s,1H),8.41 (d, J = 8.8 Hz,1H),8.12 (d, J = 8.8 Hz,1H),7.83 (d, J = 4.8 Hz,1H),5.29-5.22 (m,1H),3.97 (s,3H), 1.55 (d, J = 6.8 Hz,6H). MS (ESI, m/z):321.1 [M+H]⁺.

### Example 13: Synthesis of isopropyl

### 2-(3-cyano-1-isopropyl-1H-indazol-5-yl)isonicotinate (21)

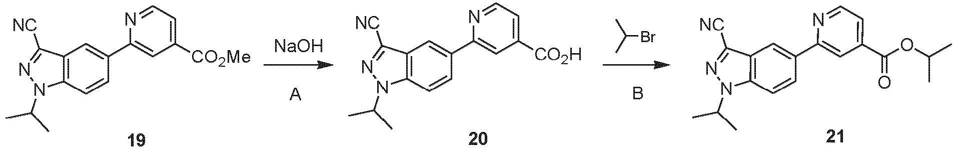

Step A: A mixture comprising compound **19** (1.0 g, 3.12 mmol), 2 M sodium hydroxide solution (15 mL), methanol (5 mL), and THF (5 mL) was stirred at room temperature for 30 minutes. Water (20 mL) was added. Ethyl acetate (50 mL) was used for extraction. The product was in the aqueous phase. The pH value of the aqueous phase was adjusted to5-6 with a 2M citric acid solution. Filtration was performed to give 2-(3-cyano-1-isopropyl-1H-indazol-5-yl)isonicotinic acid (**20**)(688 mg). The yield was 72.0%. ¹H NMR (DMSO-d₆,400 MHz): δ 8.88 (d, J = 4.8 Hz,1H),8.65 (s,1H),8.50 (s,1H),8.40 (dd, J = 1.6,9.2 Hz,1H),8.13 (d, J = 8.8 Hz,1H),7.82 (d, J = 8.8 Hz,1H),5.30-5.23 (m,1H),1.58 (d, J = 6.8 Hz,6H). MS (ESI, m/z):307.3 [M+H]⁺.

Reference can be made to Example 4 for the experimental procedure of step B, and isopropyl 2-(3-cyano-1-isopropyl-1H-indazol-5-yl)isonicotinate (**21**) was obtained. ¹H NMR (DMSO-d₆,400 MHz): δ 8.91 (dd, J = 0.8,4.8 Hz,1H),8.64 (d, J = 0.8 Hz,1H),8.48 (s,1H),8.41 (dd, J = 1.6,8.8 Hz,1H),8.12 (d, J = 8.8 Hz,1H),7.81 (dd, J = 1.6,4.8 Hz,1H),5.29-5.20 (m,2H),1.56 (d, J = 6.4 Hz,6H),1.39 (d, J = 6.4 Hz,6H). MS (ESI, m/z):349.1 [M+H]⁺.

### Example 14: Synthesis of (5-methyl-2-oxo-1,3-dioxol-4-yl)methyl

### 2-(3-cyano-1-isopropyl-1H-indazol-5-yl)isonicotinate (22)

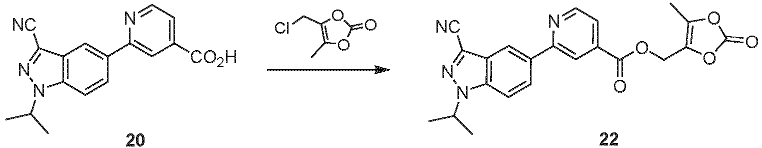

Compound **20** and 4-chloromethyl-5-methyl-1,3-dioxol-2-one were used as raw materials, and reference can be made to Example 3 for the experimental procedure for synthesizing compound **22.** ¹H NMR (DMSO-d₆,400 MHz): δ 8.93 (d, J = 5.2 Hz,1H), 8.67 (s,1H),8.53 (s,1H),8.40 (dd, J = 1.6,9.2 Hz,1H),8.12 (d, J = 9.2 Hz,1H),7.85 (dd, J = 1.6,9.2 Hz,1H),5.33 (s,2H),5.29-5.22 (m,1H),2.26 (s,3H),1.55 (d, J = 6.8 Hz,6H). MS (ESI, m/z):419.1 [M+H]⁺.

### Example 15: Synthesis of

### [2-(3-cyano-1-isopropyl-1H-indazol-5-yl)pyridine-4-carbonyl]-L-valine methyl ester (23)

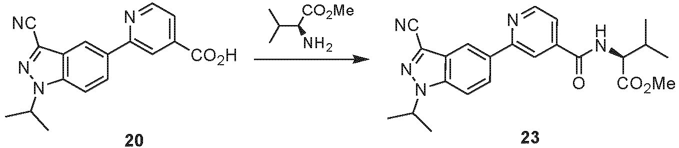

Compound **20** and L-valine methyl ester hydrochloride were used as raw materials, and reference can be made to Example 6 for the experimental procedure for synthesizing compound **23.** ¹H NMR (DMSO-d₆, 400 MHz): δ 9.10 (d, J = 7.6 Hz, 1H), 8.85 (d, J = 4.8 Hz, 1H), 8.65 (s, 1H), 8.48 (s, 1H), 8.42 (d, J = 8.8 Hz, 1H), 8.15 (d, J = 8.8 Hz, 1H), 7.77 (d, J = 5.2 Hz, 1H), 5.28-5.23 (m, 1H), 4.42-4.38 (m, 1H), 3.70 (s, 3H), 2.26-2.21 (m, 1H), 1.56 (d, J = 6.8 Hz, 6H), 1.03 (d, J = 6.8 Hz, 3H), 0.98 (d, J = 6.8 Hz, 3H). MS (ESI, m/z):539.0 [M+DMSO+ACN+H]⁺.

### Example 16: Synthesis of

### (3,7,11-trimethyldodecyl-2,6,10-trien-1-yl)2-(3-cyano-1-isopropyl-1H-indazol-5-y l)isonicotinate (24)

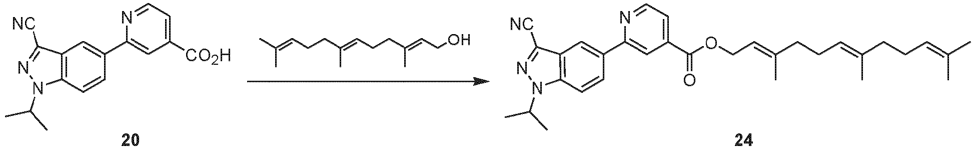

Compound **20** and 3,7,11-trimethyldodecyl-2,6,10-trien-1-ol were used as raw materials, and reference can be made to Example 8 for the experimental procedure for synthesizing compound **24.** ¹H NMR (DMSO-d₆,400 MHz): δ 8.90 (d, J = 4.8 Hz,1H), 8.63 (s,1H),8.48 (s,1H),8.38 (dd, J = 1.6,9.2 Hz,1H),8.11 (d, J = 9.2 Hz,1H),7.80 (dd, J = 1.6,5.2 Hz,1H),5.49-5.46 (m,1H),5.29-5.22 (m,1H),5.11-5.07 (m,2H),4.92-4.87 (m,2H),2.12-1.88 (m,8H),1.89-1.77 (m,6H),1.64-1.52 (m,12H). MS (ESI, m/z):511.3 [M+H]⁺.

### Example 17: Synthesis of (3,7-dimethyloctan-2,6-dien-1-yl)

### 2-(3-cyano-1-isopropyl-1H-indazol-5-yl)isonicotinate (25)

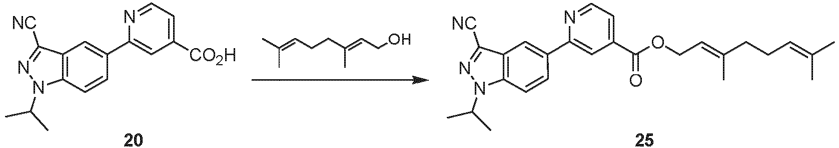

Compound **20** and 3,7-dimethyloctan-2,6-dien-1-ol were used as starting materials, and reference can be made to Example 8 for the experimental procedure for synthesizing compound **25.** ¹H NMR (DMSO-d₆,400 MHz): δ 8.90 (d, J = 4.8 Hz,1H), 8.64 (s,1H),8.48 (s,1H),8.38 (dd, J = 1.6,9.2 Hz,1H),8.11 (d, J = 9.2 Hz,1H),7.81 (dd, J = 1.6,5.2 Hz,1H),5.49-5.47 (m,1H),5.26-5.24 (m,1H),5.08-5.07 (m,1H),4.92-4.90 (m,2H),2.09-2.04 (m,4H),1.77-1.57 (m,15H). MS (ESI, m/z):443.2 [M+H]⁺.

### Example 18: Synthesis of ethyl

### {1-[(ethoxycarbonyl)oxy]}2-(3-cyano-1-isopropyl-1H-indazol-5-yl)isonicotinate(2 6)

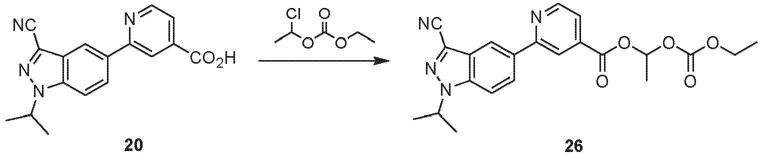

A mixture comprising compound **20** (100 mg, 0.326 mmol), potassium carbonate (90 mg, 0.651 mmol), 1-chloroethyl ethyl carbonate (75 mg, 0.492 mmol), potassium iodide (70 mg, 0.422 mmol), and DMF (3 mL) was stirred overnight at 40°C. Water (20 mL) was added. Ethyl acetate (20 mL x 3) was used for extraction. The combined organic phase was successively washed with water (15 mL x 2) and saturated brine (15 mL) and dried over anhydrous sodium sulfate. The solvent was evaporated under reduced pressure. The product was purified by column chromatography (200-300 mesh silica gel, petroleum ether: dichloromethane: triethylamine = 400: 100: 1 elution) to give ethyl {1-[(ethoxycarbonyl)oxy]} 2-(3-cyano-1-isopropyl-1H-indazol-5-yl)isonicotinate (26). ¹H NMR (DMSO-d₆,400 MHz): δ 8.94 (d, J = 5.2 Hz,1H), 8.67 (s,1H),8.52 (s,1H),8.40 (dd, J = 1.6,8.8 Hz,1H),8.11 (d, J = 8.8 Hz,1H),7.84 (dd, J = 1.6,5.2 Hz,1H),6.96 (q, J = 5.2 Hz,1H),5.29-5.22 (m,1H),4.19 (q, J = 6.8 Hz,2H),1.65 (d, J = 5.2 Hz,3H),1.56 (d, J = 6.8 Hz,6H),1.24 (d, J = 6.8 Hz,3H). MS (ESI, m/z):423.1 [M+H]⁺.

### Example 19: Synthesis of (2-acetoxy)ethyl

### 1-(3-cyano-1-isopropyl-1H-indazol-5-yl)-1H-pyrazole-4-carboxylate (27)

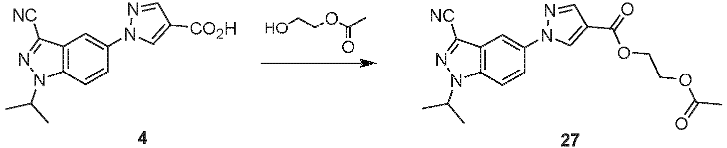

Compound **4** and ethylene glycol monoacetate were used as raw materials, and reference can be made to Example 8 for the experimental procedures for synthesizing compound 27. ¹H NMR (DMSO-d₆,400 MHz): δ 9.34 (s,1H),8.48 (d, J = 1.6 Hz,1H),8.24-8.16 (m,3H),5.29-5.23 (m,1H),4.47-4.45 (m,2H),4.35-4.33 (m,2H),2.06 (s,3H),1.55 (d, J = 6.4 Hz,6H). MS (ESI, m/z):382.5 [M+H]⁺.

### Example 20: Synthesis of (2-acetoxy)ethyl 2-(3-cyano-1-isopropyl-1H-indazol-5-yl) isonicotinate (28)

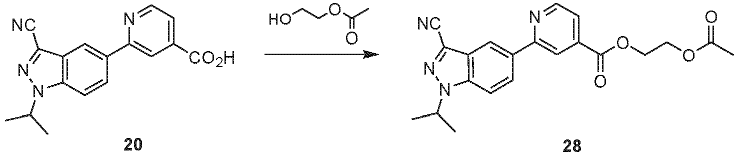

Compound 20 and ethylene glycol monoacetate were used as raw materials, and reference can be made to Example 8 for the experimental procedures synthesizing compound **28.** ¹H NMR (DMSO-d₆,400 MHz): δ 8.93 (d, J = 5.2 Hz,1H),8.64 (s,1H),8.50 (s,1H),8.38 (dd, J = 1.6,8.8 Hz,1H),8.13 (d, J = 8.8 Hz,1H),7.83 (dd, J = 1.6,5.2 Hz,1H),5.29-5.22 (m,1H),4.59-4.56 (m,2H),4.44-4.41 (m,2H),2.07 (s,3H),1.56 (d, J = 6.8 Hz,6H). MS (ESI, m/z):393.1 [M+H]⁺.

### Example 21: Synthesis of (2-methoxy)ethyl 1-(3-cyano-1-isopropyl-1H-indazol-5-yl)-1H-pyrazole-4-carboxylate (29)

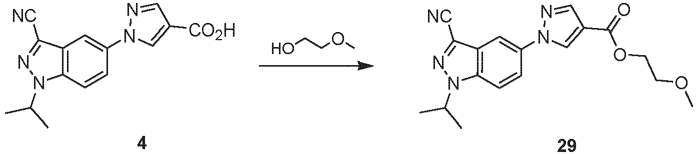

Compound **4** and ethylene glycol monomethyl ether were used as raw materials, and reference can be made to Example 8 for the experimental procedure for synthesizing compound **29.** ¹H NMR (DMSO-d₆,400 MHz): δ 9.33 (s,1H),8.49 (d, J = 1.6 Hz,1H),8.24-8.16 (m,3H),5.29-5.22 (m,1H),4.38 (t, J = 4.4 Hz,2H),3.65 (t, J = 4.4 Hz,2H),3.32 (s,3H),1.54 (d, J = 6.8 Hz,6H). MS (ESI, m/z):354.1 [M+H]⁺.

### Example 22: Synthesis of (2-methoxy)ethyl 2-(3-cyano-1-isopropyl-1H-indazol-5-yl)isonicotinate (30) and (2-methoxy)ethyl 2-(3-cyano-1-isopropyl-1H-indazol-5-yl)isonicotinate hydrobromide (31)

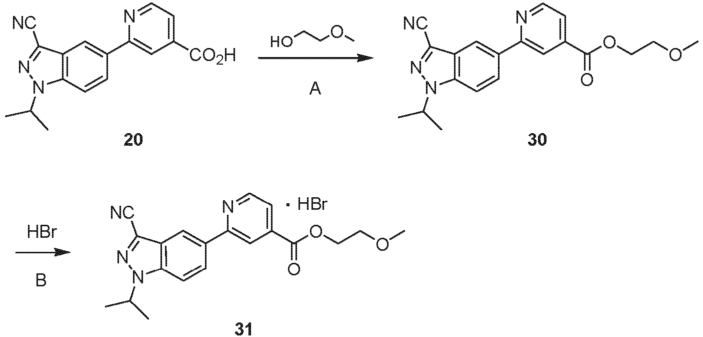

Step A: Compound **20** and ethylene glycol monomethyl ether were used as raw materials, and reference can be made to Example 8 for the experimental procedure for synthesizing compound 30. ¹H NMR (DMSO-d₆,400 MHz): δ 8.93 (d, J = 4.8 Hz,1H), 8.66 (s,1H),8.51 (s,1H),8.39 (dd, J = 1.6,9.2 Hz,1H),8.13 (d, J = 9.2 Hz,1H),7.83 (dd, J = 1.6,5.2 Hz,1H),5.29-5.23 (m,1H),4.51 (t, J = 4.4 Hz,2H),3.72 (t, J = 4.4 Hz,2H),3.33 (s,3H),1.55 (d, J = 6.4 Hz,6H). MS (ESI, m/z):365.1 [M+H]⁺.

Step B: Hydrogen bromide was charged into a dichloromethane (10 mL) solution of compound **30** (48 mg, 0.132 mmol) to make the solution acidic strongly, then dichloromethane was evaporated, and ethyl acetate/petroleum ether was used for recrystallization to obtain (2-methoxy)ethyl 2-(3-cyano-1-isopropyl-1H-indazol-5-yl)isonicotinate hydrobromide(**31**). ¹H NMR (DMSO-d₆,400 MHz): δ 8.93 (d, J = 4.8 Hz,1H), 8.65 (s,1H),8.51 (s,1H),8.39 (dd, J = 1.6,8.8 Hz,1H),8.13 (d, J = 8.8 Hz,1H),7.83 (dd, J = 1.6,5.2 Hz,1H),5.29-5.23 (m,1H),4.52-4.50 (m,2H),3.74-3.71 (m,2H),3.33 (s,3H),1.55 (d, J = 6.4 Hz,6H). MS (ESI, m/z):365.1 [M+H]⁺.

### Example 23: Synthesis of cinnamyl 1-(3-cyano-1-isopropyl-1H-indazol-5-yl)-1H-pyrazole-4-carboxylate (32)

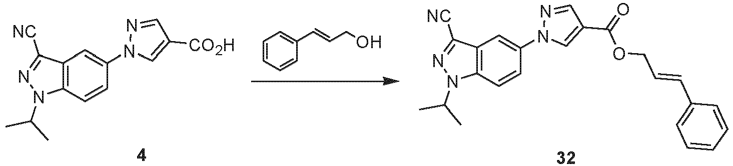

Compound **4** and cinnamyl alcohol were used as raw materials, and reference can be made to Example 8 for the experimental procedure for synthesizing compound **32.** ¹H NMR (DMSO-d₆,400 MHz): δ 9.38 (s,1H),8.49 (d, J = 1.6 Hz,1H),8.26-8.17 (m,3H),7.52-7.50 (m,2H),7.39-7.29 (m,3H),6.81 (d, J = 16.0 Hz,1H),6.54-6.46 (m,1H),5.29-5.22 (m,1H),4.95 (d, J = 5.6 Hz,2H),1.54 (d, J = 6.8 Hz,6H). MS (ESI, m/z):412.1 [M+H]⁺.

### Example 24: Synthesis of cinnamyl 2-(3-cyano-1-isopropyl-1H-indazol-5-yl) isonicotinate (33)

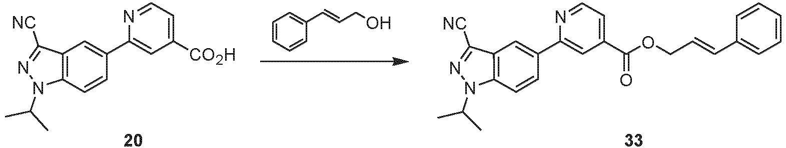

Compound **20** and cinnamyl alcohol were used as raw materials, and reference can be made to Example 8 for the experimental procedure for synthesizing compound **33.** ¹H NMR (DMSO-d₆,400 MHz): δ 8.93 (d, J = 4.8 Hz,1H),8.68 (s,1H),8.56 (s,1H),8.39 (dd, J = 1.6,8.8 Hz,1H),8.12 (d, J = 8.8 Hz,1H),7.84 (dd, J = 1.6,4.8 Hz,1H),7.54-7.52 (m,2H),7.39-7.30 (m,3H),6.86 (d, J = 16.4 Hz,1H),6.60-6.52 (m,1H),5.29-5.22 (m,1H),5.08 (d, J = 5.6 Hz,2H),1.55 (d, J = 6.6H). MS (ESI, m/z):423.1 [M+H]⁺.

### Example 25: Synthesis of (1-isobutyryloxy)ethyl 1-(3-cyano-1-isopropyl-1H-indazol-5-yl)-1H-pyrazole-4-carboxylate (34)

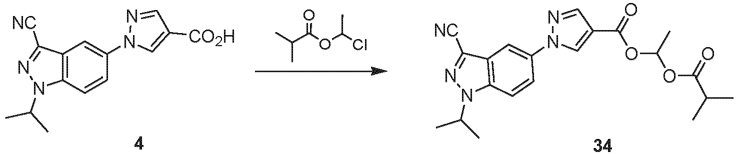

Compound **4** and 1-chloroethyl isobutyrate were used as raw materials, and reference can be made to Example 18 for the experimental procedure for synthesizing compound **34.** ¹H NMR (DMSO-d₆,400 MHz): δ 9.36 (s,1H),8.48 (s,1H),8.23-8.16 (m,3H),6.98 (q, J = 5.6 Hz,1H),5.29-5.22 (m,1H),2.62-2.55 (m,1H),1.57-1.54 (m,9H),1.12-1.10 (m,6H). MS (ESI, m/z):410.1 [M+H]⁺.

### Example 26: Synthesis of (1-isobutyryloxy)ethyl 2-(3-cyano-1-isopropyl-1H-indazol-5-yl)isonicotinate (35)

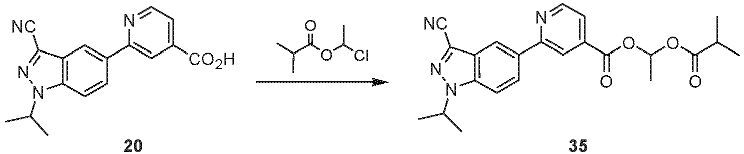

Compound **20** and 1-chloroethyl isobutyrate were used as raw materials, and reference can be made to Example 18 for the experimental procedure for synthesizing compound **35.** ¹H NMR (DMSO-d₆,400 MHz): δ 8.94 (d, J = 4.8 Hz,1H),8.66 (s,1H),8.50 (s,1H),8.39 (dd, J = 1.6,9.2 Hz,1H),8.12 (d, J = 9.2 Hz,1H),7.83 (dd, J = 1.2,4.8 Hz,1H),7.04 (q, J = 5.2 Hz,1H),5.29-5.22 (m,1H),2.65-2.58 (m,1H),1.63 (d, J = 5.2 Hz,3H),1.56 (d, J = 6.4 Hz, 6H),1.12 (d, J = 6.8 Hz,6H). MS (ESI, m/z):421.2 [M+H]⁺.

### Example 27: Synthesis of bis[2-(3-cyano-1-isopropyl-1H-indazol-5-yl) isonicotinic acid]propane-1,3-diester (36)

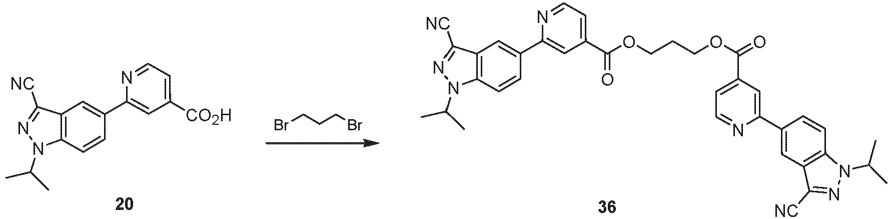

Compound **20** and 1,3-dibromopropane were used as raw materials, and reference can be made to Example 5 for the experimental procedure for synthesizing compound **36.** ¹H NMR (DMSO-d₆,400 MHz): δ 8.72 (d, J = 4.8 Hz,2H),8.32-8.31 (m,4H),8.17 (dd, J = 1.2,8.8 Hz,2H),7.98 (d, J = 8.8 Hz,2H),7.71 (d, J = 4.8 Hz,2H),5.22-5.15 (m,2H),4.62 (t, J = 6.0 Hz,4H),2.33 (t, J = 6.0 Hz,2H),1.53 (d, J = 6.4 Hz,12H). MS (ESI, m/z):653.2 [M+H]⁺.

### Example 28: synthesis of [4-(nitrooxy)]butyl 1-(3-cyano-1-isopropyl-1H-indazol-5-yl)-1H-pyrazole-4-carboxylate (38)

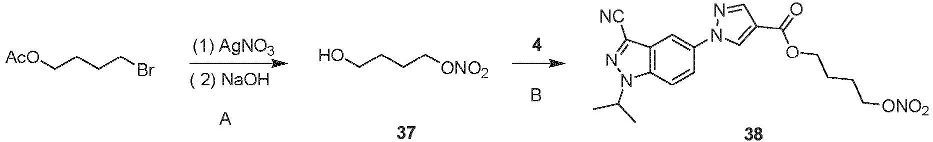

Step A: A mixture comprising 4-bromobutyl acetate (1.0 g, 5.13 mmol), silver nitrate (1.30 g, 7.65 mmol), and acetonitrile (15 mL) was stirred under reflux overnight in the dark, cooled to room temperature, and filtered to remove insolubles. Water (60 mL) was added. Ethyl acetate (30 mL x 3) was used for extraction. The combined organic phase was washed with saturated brine (20 mL), and dried over anhydrous sodium sulfate. The solvent was evaporated under reduced pressure. Then, a 2 M sodium hydroxide solution (2.5 mL) and methanol (5 mL) were added to the residue. After the addition was complete, the resulting mixture was stirred at room temperature for 2 hours. Water (20 mL) was added. Ethyl acetate (20 mL x 2) was used for extraction. The combined organic phase was washed with saturated brine (10 mL), and dried over anhydrous sodium sulfate. The solvent was evaporated under reduced pressure. The product was purified by column chromatography (200-300 mesh silica gel, petroleum ether:ethyl acetate=5:1 elution) to give 4-hydroxybutyl nitrate (**37**) (400 mg). The yield was 57.5%.

Step B: A mixture comprising compound **4** (80 mg, 0.272 mmol), compound **37** (40 mg, 0.296 mmol), DCC (84 mg, 0.407 mmol), DMAP (4 mg, 0.0327 mmol), and dichloromethane (5 mL) was stirred at room temperature overnight. Insolubles were removed by filtration. The solvent was evaporated under reduced pressure. The product was purified by column chromatography (200-300 mesh silica gel, petroleum ether: ethyl acetate = 10:1-10:3 elution) to give [4-(nitrooxy)]butyl 1-(3-cyano-1-isopropyl-1H-indazol-5-yl)-1H-pyrazole-4-carboxylate (38). ¹H NMR (DMSO-d₆,400 MHz): δ 9.31 (s,1H),8.46 (s,1H),8.23-8.16 (m,3H),5.29-5.22 (m,1H),4.61 (t, J = 6.0 Hz,2H),4.29 (t, J = 6.0 Hz,2H),1.87-1.80 (m,4H),1.54 (d, J = 6.4 Hz,6H). MS (ESI, m/z):413.3 [M+H]⁺.

### Example 29: Synthesis of [4-(nitrooxy)]butyl 2-(3-cyano-1-isopropyl-1H-indazol-5-yl) isonicotinate (39)

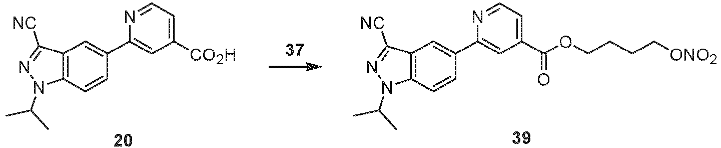

Compound **20** and compound **37** were used as raw materials, and reference can be made to Example 28 for the experimental procedurefor synthesizing compound **39.** ¹H NMR (DMSO-d₆,400 MHz): δ 8.91 (d, J = 4.8 Hz,1H),8.63 (s,1H),8.49 (s,1H),8.38 (dd, J = 1.6,9.2 Hz,1H),8.11 (d, J = 9.2 Hz,1H),7.83 (dd, J = 1.2,4.8 Hz,1H),5.29-5.22 (m,1H),4.62 (t, J = 6.0 Hz,2H),4.42 (t, J = 6.0 Hz,2H),1.89-1.86 (m,4H),1.56 (d, J = 6.4 Hz,6H). MS (ESI, m/z):424.0 [M+H]⁺.

### Example 30: Synthesis of [3-(nitrooxy)methyl]phenyl 1-(3-cyano-1-isopropyl-1H-indazol-5-yl)-1H-pyrazole-4-carboxylate (41)

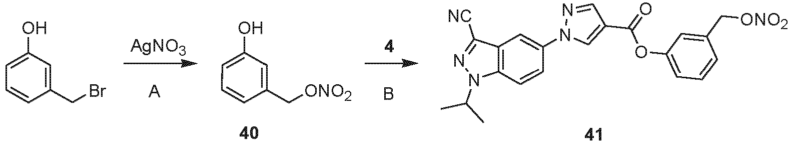

Step A: A mixture comprising m-hydroxybenzyl bromide (500 mg, 2.67 mmol), silver nitrate (500 mg, 2.94 mmol), and acetonitrile (5 mL) was stirred for 5 hours in the dark in an ice-water bath. Insolubles were removed by filtration. Water (20 mL) was added. Ethyl acetate (20 mL x 2) was used for extraction. The combined organic phase was washed with saturated brine (10 mL), and dried over anhydrous sodium sulfate. The solvent was evaporated under reduced pressure. The product was purified by column chromatography (200-300 mesh silica gel, petroleum ether: ethyl acetate=35:1 elution) to give 3-hydroxybenzyl nitrate (40) (230 mg). The yield was 50.9%.

Reference can be made to step B in Example 28 for the experimental procedure of step B, and [3-(nitrooxy)methyl]phenyl 1-(3-cyano-1-isopropyl-1H-indazol-5-yl)-1H-pyrazole-4-carboxylate (**41** was obtained. ¹H NMR (DMSO-d₆,400 MHz): δ 9.58 (s,1H),8.53 (d, J = 1.6 Hz,1H),8.40 (s,1H),8.28-8.19 (m,2H),7.57-7.53 (m,1H),7.44-7.42 (m,2H),7.37-7.35 (m,1H),5.64 (s,2H),5.30-5.23 (m,1H),1.55 (d, J = 6.4 Hz,6H). MS (ESI, m/z):447.0 [M+H]⁺.

### Example 31: Synthesis of [3-(nitrooxy)methyl]phenyl 2-(3-cyano-1-isopropyl-1H-indazol-5-yl)isonicotinate (42)

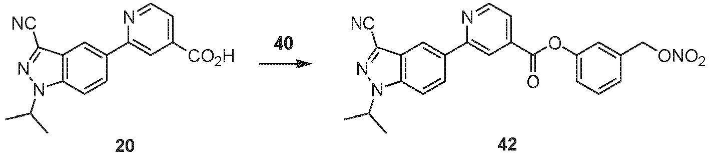

Compound **20** and compound **40** were used as raw materials, and reference can be made to step B of Example 28 for the experimental procedureforsynthesizing compound **42.** ¹H NMR (DMSO-d₆, 400 MHz): δ 9.00 (d, J = 5.2 Hz,1H),8.73 (s,2H),8.46 (dd, J = 1.6,8.8 Hz,1H),8.14 (d, J = 8.8 Hz,1H),8.01 (dd, J = 1.6,5.2 Hz,1H),7.61-7.57 (m,1H),7.53-7.52 (m,1H),7.48-7.45 (m,2H),5.66 (s,2H),5.30-5.23 (m,1H),1.56 (d, J = 6.8 Hz,6H). MS (ESI, m/z):457.9 [M+H]⁺.

### Example 32: Synthesis of [2-(nitrooxy)] ethyl 2-(3-cyano-1-isopropyl-1H-indazol-5-yl) isonicotinate (44)

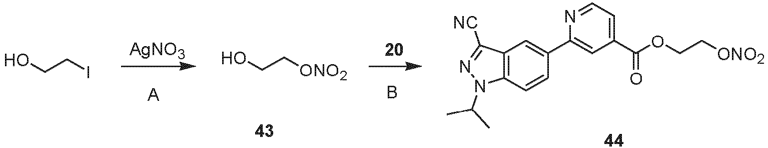

2-Iodoethanol and compound **20** were used as raw materials, and reference can be made to Example 28 for the experimental procedure forsynthesizing compound **44.** ¹H NMR (DMSO-d₆,400 MHz): δ 8.91 (d, J = 4.8 Hz,1H),8.63 (s,1H),8.50 (s,1H),8.37 (dd, J = 1.6,8.8 Hz,1H),8.11 (d, J = 8.8 Hz,1H),7.81 (dd, J = 1.2,4.8 Hz,1H),5.27-5.20 (m,1H),4.94-4.92 (m,2H),4.69-4.67 (m,2H),1.53 (d, J = 6.8 Hz,6H). MS (ESI, m/z):396.0 [M+H]⁺.

### Example 33: Synthesis of [3-(nitrooxy)] propyl 2-(3-cyano-1-isopropyl-1H-indazol-5-yl) isonicotinate (46)

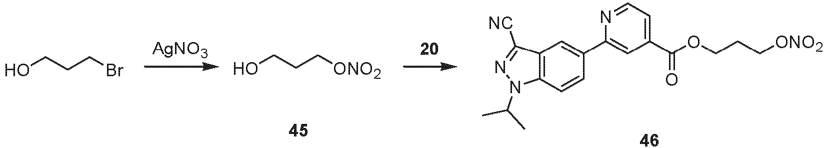

3-Bromo-l-propanol and compound 20 were used as raw materials, and reference can be made to Example 28 for the experimental procedureforsynthesizing compound **46.** ¹H NMR (DMSO-d₆,400 MHz): δ 8.91 (d, J = 5.2 Hz,1H),8.63 (s,1H),8.49 (s,1H),8.38 (dd, J = 1.6,8.8 Hz,1H),8.11 (d, J = 8.8 Hz,1H),7.83 (dd, J = 1.2,4.8 Hz,1H),5.28-5.18 (m,1H),4.56 (t, J = 6.4 Hz,2H),4.43 (t, J = 6.4 Hz,2H),1.91-1.87 (m,2H),1.54 (d, J = 6.8 Hz,6H). MS (ESI, m/z):468.5 [M+ACN+Na]⁺.

### Example 34: Experimental study on compound 22 for the treatment of hyperuricemia in rats

### 1. Experimental materials

### (1) Test drugs

Compound **22**was a light yellow powder and was triturated with 0.5% CMC-Na just prior to use to prepare a suspension having a corresponding concentration for gavage.

Febuxostat, purchased from Sigma, was triturated with 0.5% CMC-Na just prior to use to prepare a suspension having a corresponding concentration for gavage.

### (2) Animal and Feeding

### a. Animal species and source

SD rats, SPF grade, 36, male, weighing 180-200 g, purchased from Shanghai SLAC Laboratory Animal Co., Ltd., permission number: SCXK (jing) 2019-0010, quality certification number: 110324221100913432.

### b. Feeding conditions

The rats were all housed in independent ventilation cages with an air cleanliness level of 10000. The laboratory temperature: 26 ± 2° C; the relative humidity: 60%-80%; the air exchange frequency per hour: 10-15 times/hour; the light cycle: 12 (day)/12 (night) hours, 3 rats per cage.

Feed: complete pelleted feed for rats, purchased from Jiangsu Xietong Pharmaceutical Bio-engineering Co., Ltd., and its quality complying with GB14924.1-2001 "General Quality Standard for Compound Feed for Laboratory Animals".

Bedding: sterilized granule pads purchased from Jiangsu Xietong Pharmaceutical Bio-engineering Co., Ltd.

Water: purified drinking water, free drinking after acidification.

### (3) Main instruments and equipment

Varioskan LUX multifunctional microplate reader purchased from Thermo, USA; BS210S precision electronic balance (0.1 mg-10 g) purchased from Sartorius, Germany; FEJ-200 electronic balance (0.1-200 g)purchased from Fuzhou FuriWeighting Electronics Co., Ltd.; Pacific TII+Genpure XCAD PLUS UV/TOC/UF pure water ultrapure water system purchased from Thermo, USA.

### (4) Main reagents

Uric acid assay kit (phosphotungstic acid reduction method), batch number: 20220305, purchased from NanJing Jiancheng Bioengineering Institute; Potassium oxonate, article number: 00164, batch number:GR4VI-RK, purchased from Tokyo Chemical Industry Co., Ltd., Japan (TCI); carboxymethyl cellulose sodium (CMC-Na), batch number: 20170810, chemically pure, purchased from Sinopharm chemical reagent Co., Ltd.

### 2. Experimental methods

### (1) Grouping

36 male SD rats weighed approximately 200-230 g after one week of adaptation. They were randomly divided into 6 groups according to the body weight, with 6 rats in each group, namely: (1) normal group (0.5% CMC-Na), (2) model group (0.5% CMC-Na), (3) Febuxostat 1 mg/kg, (4) Febuxostat 2 mg/kg, (5) compound **22**, 1.45 mg/kg, (6) compound **22,** 2.9 mg/kg. The drugs in each group were prepared into a corresponding concentration suspension, and the administration volume was 0.5 mL/100 g.

### (2) Model establishment, dosing regimen and detection indicators

After the rats in each group were purchased and adapted to feeding, they were fasted for 12 h, and intraperitoneally injected with potassium oxonate at a dose of 300 mg/kg to establish the model. After 0.5 h of model establishment, each test drug group was gavaged once. Blood was collected from the retro-orbital venous plexus before and 1, 3, and 5 h after potassium oxonate injection. The samples were centrifuged at 3500 rpm for 10 min. 30 µL of serum was collected to determine the uric acid level at each time point.

### (3) Data processing and statistical methods

The measurement data of each test were expressed as (mean) ±standard deviation). The comparison between groups used ANOVA-Dunnett T test to examine the significance. P<0.05 was used as the significance index, and P<0.01 was used as the extremely significant index.

### 3. Experimental results

The results are shown in Table 1. Compared with the normal group, the serum uric acid level in the potassium oxonate model group increased significantly at 1, 3, and 5 h after modeling (P<0.05). Compared with the model group at the same time point, Febuxostat 1 mg/kg and 2 mg/kg can both significantly reduce the serum uric acid level at 1, 3, and 5 h after modeling (P<0.01). Compared with the model group at the same time point, the 1.45 mg/kg group of compound **22** can significantly reduce the serum uric acid level at 1 h after modeling(P<0.05). The 2.9 mg/kg group of compound **22**can significantly reduce the serum uric acid level at 1 and 5 h after modeling (P<0.05).

**Table 1. Effect on serum uric acid level in potassium oxonate-induced hyperuricemic rats following administration (x̅ ±s)**

| Group | Dose (mg/kg) | Number of animals | Uric acid (µmol/L) | | | |
|---|---|---|---|---|---|---|
| | | | 0 h | 1h | 3 h | 5 h |
| normal group | - | 6 | 54.6±12.4 | 84.6±5.5 | 65.2±13.3 | 75.0±5.4 |
| model group | - | 6 | 56.7±8.6 | 115.4±17.9^{##} | 88.8±20.8^{#} | 85.4±11.4^{#} |
| Febuxostat | 1 | 6 | 52.6±8.4 | 78.0±17.5** | 63.3±13.8** | 59.0±9.8** |
| | 2 | 6 | 52.4±9.7 | 40.6±10.6** | 50.1±10.7** | 61.4±7.1** |
| compound 22 | 1.45 | 6 | 64.0±13.1 | 88.1±17.8* | 86.5±11.5 | 78.1±11.1 |
| | 2.90 | 6 | 66.4±7.0 | 88.3±11.9* | 77.1±13.2 | 70.9±6.3* |

| | | | | | | |
|---|---|---|---|---|---|---|
| Note: ^{#}P< 0.05, ^{##}P< 0.01, compared with the normalgroup at the same time point; * P<0.05, **P<0.01, compared with the model group at the same time point. | | | | | | |

### Example 35: Experimental study on compounds 13, 38 and 41 for the treatment of hyperuricemia in rats

### 1. Experimental materials

### (1) Test drugs

Compound **13**was a light yellow powder, and compounds **38** and **41** are pseudo-white powders, which are triturated with 0.5% CMC-Na just prior to use to prepare a 0.4 mg/mL suspension for gavage.

Febuxostat, purchased from Sigma, was triturated with 0.5% CMC-Na just prior to use to prepare a 0.4 mg/mL suspension for gavage.

### (2) Animal and feeding

### a. Animal species and source

SD rats, SPF grade, 36, male, weighing 180-200 g, purchased from Shanghai SLAC Laboratory Animal Co., Ltd., permission number: SCXK (jing) 2019-0010, quality certification number: 110324221100913432.

### b. Feeding conditions

The rats were all housed in independent ventilation cages with an air cleanliness level of 10000. The laboratory temperature: 26 ±2° C; the relative humidity: 60%-80%; the air exchange frequency per hour: 10-15 times/hour; the light cycle: 12 (day)/12 (night) hours, 3 rats per cage.

Feed: complete pelleted feed for rats, purchased from Jiangsu Xietong Pharmaceutical Bio-engineering Co., Ltd., and its quality complying with GB14924.1-2001 "General Quality Standard for Compound Feed for Laboratory Animals".

Bedding: sterilized granule pads purchased from Jiangsu Xietong Pharmaceutical Bio-engineering Co., Ltd.

Water: purified drinking water, free drinking after acidification.

### (3) Main instrumentsand equipment

Varioskan LUX multifunctional microplate reader purchased from Thermo, USA; BS210S precision electronic balance (0.1 mg-10 g) purchased from Sartorius, Germany; FEJ-200 electronic balance (0.1-200 g) purchased from Fuzhou FuriWeightingElectronics Co., Ltd.; Pacific TII+Genpure XCAD PLUS UV/TOC/UF pure water ultrapure water system purchased from Thermo, USA.

### (4) Main reagents

Uric acid assay kit (phosphotungstic acid reduction method), batch number: 20230224, purchased from NanJing Jiancheng Bioengineering Institute; Potassium oxonate, article number: 00164, batch number:T6GKM-TA, purchased from Tokyo Chemical Industry Co., Ltd., Japan (TCI); carboxymethyl cellulose sodium (CMC-Na), batch number: 20170810, chemically pure, purchased from Sinopharm chemical reagent Co., Ltd.

### 2. Experimental methods

### (1) Grouping

36 male SD rats weighed approximately 220-240 g after one week of adaptation. They were randomly divided into 6 groups according to the body weight, with 6 rats in each group, namely: (1) normal group (0.5% CMC-Na), (2) model group (0.5% CMC-Na), (3) Febuxostat 2 mg/kg, (4) compound **13,** 2 mg/kg, (5) compound **38,** 2 mg/kg, and (6) compound **41,** 2 mg/kg. The drugs in each group were prepared into a corresponding concentration suspension, and the administration volume was 0.5 mL/100 g.

### (2) Model establishment, dosing regimen and detection indicators

After the rats in each group were purchased and adapted to feeding, they were fasted for 12 h, and. intraperitoneally. injected with potassium oxonate at a dose of 300 mg/kg to establish the model. 0.5 h after modeling, each test drug group was gavaged once. The drug was administered continuously for 3 days. On day 3, blood samples was collected from the retro-orbital venous plexus before and 1, 3, and 5 h after potassium oxonate injection. The samples were centrifuged at 3500 rpm for 10 min, and 30 µL of serum was collected to determine the uric acid level at each time point.

### (3) Data processing andstatistical methods

The measurement data of each test were expressed as (mean) ±standard deviation). The comparison between groups used ANOVA-Dunnett T test to examine the significance. P<0.05 was used as the significance index, and P<0.01 was used as the extremely significant index.

### 3. Experimental results

The results are shown in Table 2. Compared with the normal group, the serum uric acid level in the potassium oxonatemodel group increased significantly at 1, 3, and 5 h after modeling (P<0.05). Compared with the model group at the same time point, the Febuxostat group can significantly reduce the serum uric acid level at 1, 3, and 5 h after modeling (P<0.01). Compared with the model group at the same time point, compound **13**can significantly reduce the serum uric acid level at 1, 3, and 5 h after modeling (P<0.01 or P<0.05). Compound **38** can significantly reduce the serum uric acid level at 1, 3, and 5 h after modeling (P<0.01 or P<0.05). Compound **41**can significantly reduce the serum uric acid level at 3 and 5 h after modeling (P<0.01).

**Table 2. Effect on serum uric acid level in potassium oxonate-induced hyperuricemic rats following administration (x̅ ±s)**

| Group | Dose (mg/kg) | Number of animals | Uric acid (µmol/L) | | | |
|---|---|---|---|---|---|---|
| | | | 0 h | 1 h | 3 h | 5 h |
| Normal group | - | 6 | 46.8±6.0 | 52.0±9.1 | 55.7±6.4 | 56.0±9.4 |
| Model group | - | 6 | 49.3±12.3 | 86.6±11.1^{##} | 120.8±20.2^{##} | 107.2±27.2^{##} |
| Febuxostat | 2 | 6 | 39.2±6.2 | 3 4.4±7.1** | 31.2±7.6** | 24.7±4.7** |
| Compound **13** | 2 | 6 | 40.7±4.4 | 42.1±10.6** | 66.0±10.7** | 75.0±7.1* |
| Compound **38** | 2 | 6 | 51.7±5.9 | 67.5±19.3* | 69.0:1:24.7** | 51.7±6.7** |
| Compound **41** | 2 | 6 | 50.6±7.3 | 74.1±16.5 | 72.2±20.2** | 55.4±14.9** |

| | | | | | | |
|---|---|---|---|---|---|---|
| Note: ^{##}< P < 0.01, compared with the normal group at the same timepoint; *P<0.05, **P<0.01, compared with the model group at the same time point. | | | | | | |

### Example 36: Pharmacokinetics of the compounds in SD rats

### 1. Experimental materials

### (1) Test drugs

Preparation of compound stock solution: An appropriate amount of compound solid powder was be weighed and added in a certain amount of DMSO, and vortex ultrasound to obtain a 10 mg/mL stock solution.

Preparation of test compound for gavage: respectively taking an appropriate amount of the compound stock solution, adding a certain amount of Solutol HS15 solution, vortexing for 1 minute, then adding a certain amount of physiological saline, and fully mixing to obtain a 1 mg/mL solution.

Preparation of test compound for intravenous injection: respectively taking an appropriate amount of the compound stock solution, adding a certain amount of Solutol HS15 solution, vortexing for 1 minute, then adding a certain amount of physiological saline, and fully mixing until uniform, so as to obtain a 0.5 mg/mL solution.

### (2) Experimental animal

SD rats, male, SPF grade, 6-8 weeks old, purchased from JH Laboratory Animal Co. LTD. Permission number: SCXK (SH) 2017-0012, SCXK (SH) 2022-0009, certification number: 20170012022154, 20220009005149.

### 2. Experimental methods

### (1) Dose and administration

The experimental animals were fasted overnight before gavage administration, and were given food 4 hours after administration, with free access to water. Each test compound was divided into two groups, namely intravenous administration group and oral administration group. The specific dosage and method of administration are shown in Table 3 below.

**Table 3. Dose and administration of compounds to SD rats**

| **Group** | **Dose of administration (mg/kg)** | **Volume of administration (mL/kg)** | **Concentration (mg/mL)** | **Route of administration** |
|---|---|---|---|---|
| Intravenous administration group | 1 | 2 | 0.5 | intravenous injection |
| Oral administration group | 10 | 10 | 1 | oral |

### (2) Experimental procedure

Blood samples (150 µL/sample) were collected from the jugular vein of SD rats before administration and at 5 min (only intravenous administration group), 15 min, 30 min, 1 h, 2 h, 4 h, 8 h and 24 h after administration, respectively. The samples were placed in centrifuge tubes containing anticoagulant sodium heparin and centrifuged at 4°C, 2000 g for 5 minutes to separate plasma. The plasma samples were analyzed by LC/MS/MS to detect the concentration of each test compound.

### (3) Pharmacokinetic analysis

Non-compartmental model-related parameters were calculated by WinNonlin^{®} Professional software.

### 3. Experimental results

The pharmacokinetic parameters of the tested compounds in SD rats obtained according to the method above are shown in Table 4. The pharmacokinetic parameters of the compounds of the present invention are good and the bioavailability is high.

**Table 4. Pharmacokinetic parameters of each compound in SD rats after oral or intravenous administration**

| **Gavage administration - 10 mg/kg** | | | | | |
|---|---|---|---|---|---|
| **Compound number** | **t_{1/2} (h)** | **Tₘₐₓ (h)** | **Cₘₐₓ (ng/mL)** | **AUC_{INF} (hr*ng/mL)** | **MRT_{INF} (h)** |
| **2** | 2.91 | 0.667 | 4777 | 30130 | 4.78 |
| **5** | 3.78 | 1.17 | 6750 | 25136 | 3.17 |
| **7** | 3.27 | 0.500 | 2920 | 12920 | 3.84 |
| **19** | 2.33 | 0.667 | 6170 | 25295 | 2.86 |
| **26** | 2.52 | 0.917 | 5523 | 30972 | 3.57 |
| **28** | 2.93 | 2.17 | 3147 | 15774 | 3.55 |
| **31** | 2.75 | 2.00 | 4310 | 20872 | 3.39 |
| **39** | 3.03 | 0.500 | 3897 | 18301 | 3.90 |
| **42** | 2.91 | 1.58 | 1513 | 8637 | 4.12 |

| **Intravenous injection administration - 1 mg/kg** | | | | | |
|---|---|---|---|---|---|
| **Compound number** | **t_{1/2} (h)** | **Tₘₐₓ (h)** | **Cₘₐₓ (ng/mL)** | **AUC_{INF} (hr*ng/mL)** | **MRT_{INF} (h)** |
| **2** | 6.12 | 0.139 | 843 | 1402 | 5.05 |
| **5** | 7.45 | 0.250 | 554 | 1487 | 8.38 |
| **7** | 8.60 | 0.194 | 338 | 674 | 7.65 |
| **19** | 8.78 | 0.250 | 518 | 776 | 6.46 |
| **26** | 7.11 | 0.083 | 1967 | 1294 | 2.47 |
| **28** | 8.21 | 0.083 | 712 | 947 | 4.31 |
| **31** | 9.03 | 0.083 | 904 | 1011 | 4.75 |
| **39** | 9.92 | 0.083 | 714 | 886 | 7.16 |
| **42** | 6.44 | 0.083 | 2.82 | 767 | 3.37 |

## Claims

1. A compound as represented by general formula (I) or a pharmaceutically acceptable salt thereof, 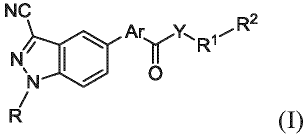 wherein,
R is C₁₋₆ alkyl, substituted C₁₋₆ alkyl, C₃₋₆ cycloalkyl, substituted C₃₋₆ cycloalkyl, C₃₋₆ heterocycloalkyl, or substituted C₃₋₆ heterocycloalkyl; wherein the substituent in each group involved in R is one or more selected fromdeuterium, cyano, nitro, halo, C₁₋₆ alkyl, C₁₋₆ alkoxy, C₃₋₆ cycloalkyl, or C₃₋₆ heterocycloalkyl;
Ar is a substituted or unsubstituted group of: 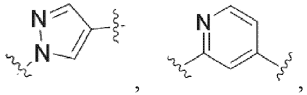 or 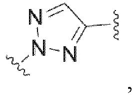 and the substituent in the Ar group is one or more selected fromdeuterium, hydroxyl, halo, C₁₋₄alkyl, or C₁₋₄alkoxy;
Y is O or NR³,
R¹ is a linking bond or substituted or unsubstituted C₁₋₆ alkylene or substituted or unsubstituted C₂₋₁₂ alkenylene, and the substituent in the R¹ group is one or more selected from deuterium, hydroxyl, amino, cyano, halo, C₁₋₄ alkyl, or C₁₋₄ alkoxy;
R² is one or more of hydrogen, nitrooxy, carboxyl, or the following substituted or unsubstituted groups: dioxol-2-one, C₄₋₁₂fused heteroarylcyclyl, C₄₋₁₆ fused heteroarylcyclylpyrazolylcarbonyloxy, C₄₋₁₆ fused heteroarylcyclylpyridylcarbonyloxy, C₄₋₁₆ fused heteroarylcyclyltriazolylcarbonyloxy, C₂₋₆ ester, pyridyl, phenyl, C₁₋₆ alkoxy, C₂₋₂₀ alkenyl, C₂₋₂₀ alkynyl, C₂₋₈ alkylcarbonyloxy, or C₂₋₈ alkoxycarbonyloxy, and the substituent in the R² group is one or more selected fromdeuterium, hydroxyl, amino, cyano, halo, C₁₋₆ alkyl, halo C₁₋₆ alkyl, nitrooxy-substituted C₁₋₆ alkyl, or C₁₋₆ alkoxy; and
R³ is hydrogen or C₁₋₆ alkyl.

2. The compound or the pharmaceutically acceptable salt thereof according to claim 1, wherein the compound is selected from compounds as represented by general formula (II), (III), or (IV):

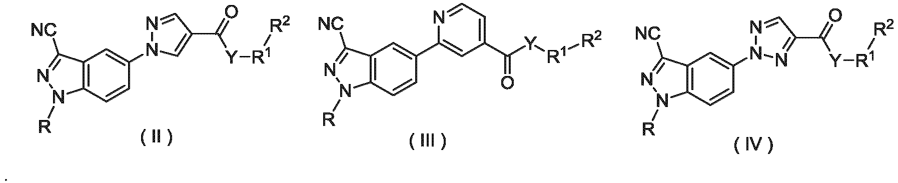

3. The compound or the pharmaceutically acceptable salt thereof according to claim 1, wherein Y is O or NH, R is C₃₋₆ alkyl, substituted C₁₋₆ alkyl, C₃₋₆ cycloalkyl, substituted C₃₋₆ cycloalkyl, C₃₋₆ heterocycloalkyl, or substituted C₃₋₆ heterocycloalkyl; wherein the substituent in each group involved in R is one or more selected fromdeuterium, cyano, nitro, halo, C₁₋₅ alkyl, C₁₋₅ alkoxy, or C₃₋₆ cycloalkyl.

4. The compound or the pharmaceutically acceptable salt thereof according to claim 1, wherein R is C₃₋₆ alkyl, substituted C₁₋₆ alkyl, C₃₋₆ cycloalkyl, substituted C₃₋₆ cycloalkyl, tetrahydrofuran, substituted tetrahydrofuran, tetrahydrothiophene, substituted tetrahydrothiophene, tetrahydropyrrole, or substituted tetrahydropyrrole; wherein the substituent in each group involved in R is one or more selected from deuterium, cyano, nitro, halo, C₁₋₅ alkyl, C₁₋₅ alkoxy, or C₃₋₆ cycloalkyl.

5. The compound or the pharmaceutically acceptable salt thereof according to claim 1, wherein R¹ is a linking bond or substituted or unsubstituted C₁₋₄ alkylene or substituted or unsubstituted C₄₋₁₂ alkenylene, and the substituent in the R¹ group is one or more selected fromdeuterium, amino, cyano, halogen, or C₁₋₄ alkoxy.

6. The compound or the pharmaceutically acceptable salt thereof according to claim 1, wherein R² is one or more of hydrogen, nitrooxy, carboxyl, or the following substituted or unsubstituted groups: dioxol-2-one, indazolyl, quinolinyl, isoquinolinyl, indolyl, benzofuranyl, purinyl, indazolylpyrazolylcarbonyloxy, quinolinylpyrazolylcarbonyloxy, isoquinolinylpyrazolylcarbonyloxy, indolylpyrazolylcarbonyloxy, benzofuranylpyrazolylcarbonyloxy, purinylpyrazolylcarbonyloxy, indazolylpyridylcarbonyloxy, quinolinylpyridylcarbonyloxy, isoquinolinylpyridylcarbonyloxy, indolylpyridylcarbonyloxy, benzofuranylpyridylcarbonyloxy, purinylpyridylcarbonyloxy, indazolyltriazolylcarbonyloxy, quinolyltriazolylcarbonyloxy, isoquinolyltriazolylcarbonyloxy, indolyltriazolylcarbonyloxy, benzofuranyltriazolylcarbonyloxy, purinyltriazolylcarbonyloxy, C₂₋₆ ester, pyridyl, phenyl, C₁₋₆ alkoxy, C₆₋₂₀ alkenyl, C₆₋₂₀ alkynyl, C₂₋₈ alkylcarbonyloxy, or C₂₋₈ alkoxycarbonyloxy, and the substituent in the R² group is one or more selected from deuterium, hydroxyl, amino, cyano, halo, C₁₋₆ alkyl, halo C₁₋₆ alkyl, nitrooxy-substituted C₁₋₆ alkyl, or C₁₋₆ alkoxy.

7. The compound or the pharmaceutically acceptable salt thereof according to claim 5, wherein R² is one or more of hydrogen, nitrooxy, carboxyl, or the following substituted or unsubstituted groups: dioxol-2-one, indazolylpyrazolylcarbonyloxy, indazolylpyridylcarbonyloxy, indazolyltriazolylcarbonyloxy, indolylpyrazolylcarbonyloxy, indolylpyridylcarbonyloxy, indolyltriazolylcarbonyloxy, C₂₋₆ester, pyridyl, phenyl, C₁₋₆alkoxy, C₆₋₂₀alkenyl, C₂₋₈alkylcarbonyloxy, or C₂₋₈alkoxycarbonyloxy, and the substituent in the R² group is one or more selected fromdeuterium, hydroxyl, amino, cyano, halo, C₁₋₆ alkyl, nitrooxy-substituted C₁₋₆ alkyl, or C₁₋₆ alkoxy.

8. The compound or the pharmaceutically acceptable salt thereof according to claim 1, wherein the compound is selected from:

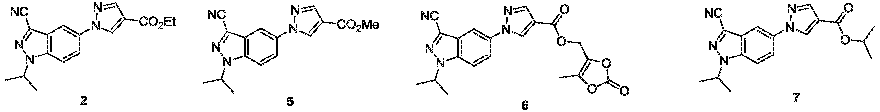

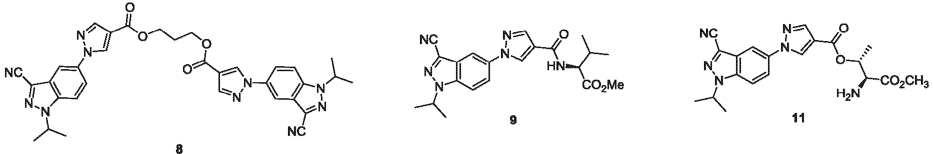

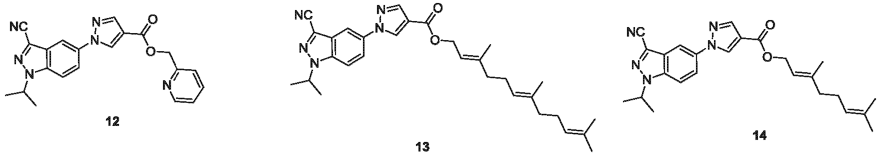

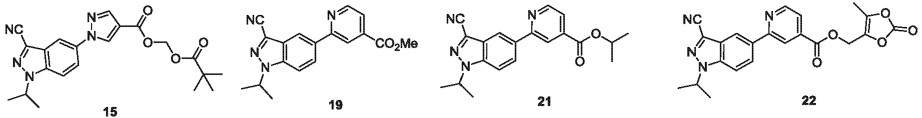

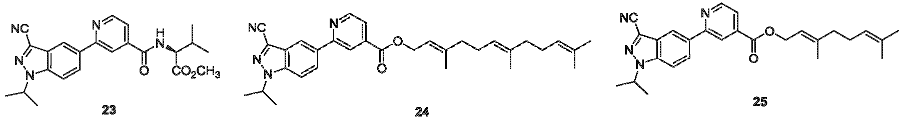

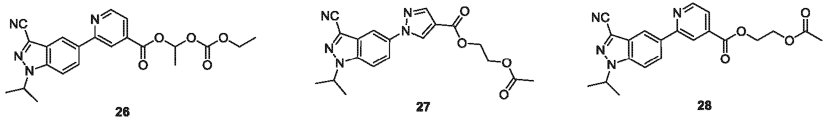

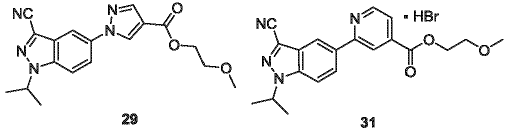

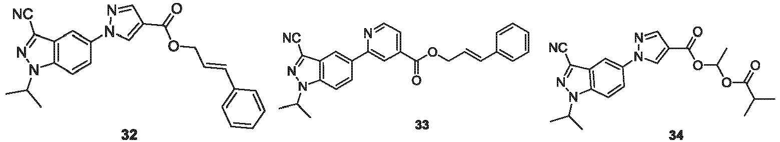

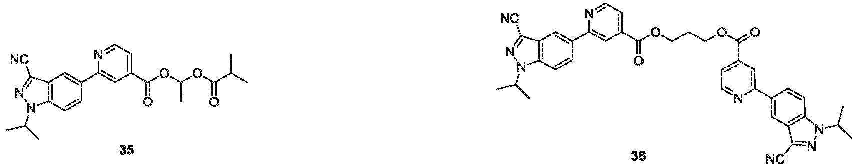

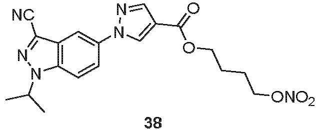

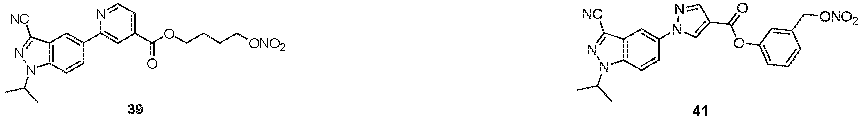

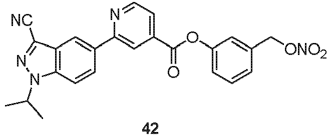

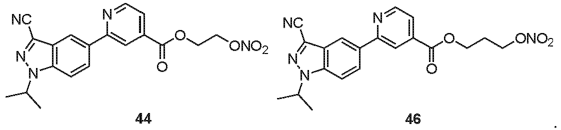

9. A pharmaceutical composition, comprising the compound or the pharmaceutically acceptable salt thereof of claim 1 as an active substance and pharmaceutically acceptable excipients.

10. Use of the compound or the pharmaceutically acceptable salt thereof of claim 1 in the preparation of an anti-gout medicament or an anti-hyperuricemia medicament.
